(19) Europäisches Patentamt
European Patent Office
Office européen des brevets

(11) **EP 2 981 572 B1**

(12) **EUROPÄISCHE PATENTSCHRIFT**

(45) Veröffentlichungstag und Bekanntmachung des Hinweises auf die Patenterteilung:
**25.10.2017 Patentblatt 2017/43**

(21) Anmeldenummer: **14719643.0**

(22) Anmeldetag: **27.03.2014**

(51) Int Cl.:
*C08J 11/12* (2006.01)　　*C10G 1/10* (2006.01)
*C08L 23/02* (2006.01)　　*C08L 23/08* (2006.01)
*C08L 23/10* (2006.01)

(86) Internationale Anmeldenummer:
**PCT/EP2014/056166**

(87) Internationale Veröffentlichungsnummer:
**WO 2014/161767 (09.10.2014 Gazette 2014/41)**

(54) **VERFAHREN ZUM ABBAU VON SYNTHETISCHEN POLYMEREN UND EINE VORRICHTUNG ZU DESSEN DURCHFÜHRUNG**

METHOD FOR THE DEGRADING OF SYNTHETIC POLYMERS AND DEVICE FOR CARRYING OUT SAID METHOD

PROCÉDÉ DE DÉCOMPOSITION DE POLYMÈRES SYNTHÉTIQUES ET DISPOSITIF PERMETTANT LA MISE EN OEUVRE DU DIT PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.04.2013 DE 102013205996**

(43) Veröffentlichungstag der Anmeldung:
**10.02.2016 Patentblatt 2016/06**

(73) Patentinhaber: **Achim Methling Josef Ranftl GBR 82256 Fürstenfeldbruck (DE)**

(72) Erfinder: **METHLING, Achim A-1110 Wien (AT)**

(74) Vertreter: **Maikowski & Ninnemann Patentanwälte Partnerschaft mbB Postfach 15 09 20 10671 Berlin (DE)**

(56) Entgegenhaltungen:
**EP-A1- 2 161 299　　EP-A2- 0 276 081
WO-A1-00/66656　　DE-A1- 19 722 586
FR-A1- 2 931 482**

**Beschreibung**

Die vorliegende Erfindung betrifft ein Verfahren zum Abbau von synthetischen Polyolefinen gemäß Anspruch 1, eine Vorrichtung bzw. Anlage zur Durchführung dieses Verfahrens gemäß Anspruch 16 und ein mit dem Verfahren hergestelltes Produktöl gemäß Anspruch 23.

**Beschreibung**

Kunststoffe auf der Basis von synthetischen Polymeren, wie zum Beispiel Polyolefinen, sind aus der heutigen Lebenswelt nicht mehr wegzudenken. Im großen Maßstab werden Polyolefine wie Polyethylen (PE) oder Polypropylen (PP) als Verpackungsmaterialien in der Konsumgüterindustrie verwendet. Obwohl große Anstrengungen zur Wiederverwertung bzw. Recycling von derartigen Kunststoffen unternommen werden, besteht nach wie vor der Bedarf an einer effizienten Ausnutzung bzw. Wiedernutzung der in den Verpackungen verarbeiteten Polyolefine.

So wurden z.B. in den Ländern der europäischen Union, Norwegen und der Schweiz 51,3% (12,8 Mt) des 2008 erzeugten Endverbraucher-Kunststoffabfalls wiederverwertet und die verbleibende Menge (12,1 Mt) wurde zum größten Teil in Mülldeponien entsorgt oder zu einem geringerem Anteil Verbrennungsanlagen ohne Energiegewinnung zugeführt. Der wieder gewonnene Plastikabfall wurde entweder zur Energiegewinnung (7,4 Mt oder 30% des Endverbraucher-Kunststoffabfalls) oder dem Recycling (5,3 Mt oder 21,3% des Endverbraucher-Kunststoffabfalls) zugeführt. Die Energiegewinnung in Müllverbrennungsanlagen erreichte 6,8 Megatonnen an Kunststoffabfall (das entspricht 27,3% des Endverbraucher-Abfalls). Die Energiegewinnung durch Verbrennen von Plastikmüll in Müllverbrennungsanlagen wird auch als End-of-Life-Anwendung bezeichnet. An erster Stelle steht dabei die Entsorgung.

Zwischen den Jahren 2006 und 2008 stieg der Anteil der Wiederverwendung von Kunststoffabfällen insgesamt an, wobei der Anteil des Recyclings stärker anstieg als der Anteil zur Energiegewinnung.

Der Anteil an Energiegewinnung und Recycling von Kunststoffabfällen unterscheidet sich stark zwischen den europäischen Ländern. So werden in Norwegen, Schweden, Deutschland, Dänemark, Belgien und Schweiz Energiegewinnungsraten zwischen 85% und 99,5% erreicht. Dies liegt insbesondere in den umfangreichen Restriktionen der Entsorgung von Kunststoffabfällen in Mülldeponien in diesen Ländern begründet.

Wie erwähnt, wird ein großer Anteil der Kunststoffabfälle nicht nur Müllverbrennungsanlagen zum Zwecke der Energieverwendung zugeführt, sondern ebenfalls recycelt. Die Recyclingrate hängt stark von den verschiedenen Kunststoffarten ab. So werden 40% der Flaschen und industriellen Folien sowie über 90% von Kisten und Boxen recycelt. Weniger als 10% der verbleibenden gemischten Kunststoffe hingegen werden in der EU recycelt. Die Gesamtrecyclingrate von Verpackungsmaterialien in Haushalt und kommerziell betrug in der EU 2008 29%, und die Wiederverwertungsrate wurde auf ca. 58% geschätzt.

In den Recyclinganlagen für Polymerkunststoffe werden die Altkunststoffe in Ballenform angeliefert, welche mindestens 92 % Polymerkunststoffe enthalten. Diese Ballen durchlaufen die Arbeitsschritte Ballenöffnen, Schreddern, Abtrennung metallischer/mineralischer Bestandteile, Abtrennung biogener Stoffe, Abtrennung von ligninhaltigen Stoffen, Abtrennung von chlorierten Kunststoffen, Waschen, Trocknen und Trennung in Polyethylenanteile und Polypropylenanteile. Die reinen Polyethylenanteile und die reinen Polypropylenanteile werden bei qualifizierten Recyclingunternehmen zu Regranulaten aufbereitet und dann dem industriellen Zyklus wieder zugeführt. Die nicht sortenrein trennbaren Altkunststoffabfälle, welche nicht in Recyclat-prozesse passen, aber trotzdem einen Polyolefinanteil von über 90 % enthalten, werden derzeit gar nicht oder nur ungenügend verwertet.

Ein generelles Problem bei der Wiederverwertung von Kunststoffabfällen besteht in der Zusammensetzung der verwendeten Kunststoffe. So bestehen die im Verbraucherbereich verwendeten Kunststoffprodukte, wie zum Beispiel Flaschen, typischerweise aus verschiedenen Polyethylenen, wie zum Beispiel Hart-Polyethylen (HDPE), oder Polyethylenterephthalat (PET), wobei die aus reinen Polymeren bestehenden Kunststoffabfälle bereits jetzt schon zu einem großen Anteil recycelt und wiederverwendet werden.

Problematisch ist hingegen das Recycling von Kunststoffabfällen, die aus Gemischen von verschiedenen Polymeren bestehen, wie zum Beispiel Polyethylen oder Polypropylen. LDEP (low density polyethylen), HDPE (high density polyethylen) und PP (Polypropylen) bilden ungefähr die Hälfte der globalen Kunststoffabfälle.

Auch wenn die Wiederverwertung von Kunststoffabfällen in Form von Recycling oder Verbrennung zur Energiegewinnung in den letzten Jahren zugenommen hat, besteht nach wie vor ein Bedarf an verbesserten Verfahren zur Verwertung von Kunststoffabfällen, insbesondere da der weltweite Verbrauch durch die Schwellenländer enorm ansteigen wird. Auch ist eine sinnvolle Wiederverwertung von Kunststoffabfällen notwendig, da die üblicherweise für die Herstellung von Kunststoffen verwendeten Ausgangsprodukte aus Erdöl bzw. Erdgas begrenzt sind.

So wurden bereits in der Vergangenheit verschiedene Ansätze zum Abbau bzw. Umwandlung von Kunststoffabfällen in die den Kunststoffen zugrundeliegenden Kohlenwasserstoffe beschrieben.

So ist aus der US 4,584,421 ein Verfahren bekannt, in welchem Kunststoff bzw. Plastikschnitzel in Gegenwart eines geeigneten Katalysators, wie zum Beispiel einen Zeolithkatalysator, einer thermischen Dekomposition bzw. einem thermischen Abbau unterworfen wird und dabei in flüssiges Kohlenwasserstofföl umgewandelt wird. In diesem Verfahren werden die Plastikschnitzel zunächst geschmolzen und anschließend bei einer Temperatur zwischen 440° und 470°C thermisch abgebaut, und die gasförmigen Produkte des thermischen Abbaus werden in einem Katalysatorbett mit Temperaturen zwischen 350° bis 470° Celsius eingeführt. Dabei erfolgt eine zweite thermische Zersetzung und die Herstellung eines Öles bestehend aus niederen Kohlenwasserstoffen, insbesondere $C_5$ bis $C_{29}$ Kohlenwasserstoffen. Das Molekulargewicht der aus den Plastikschnitzeln hergestellten niederen Kohlenwasserstoffen liegt in einem Bereich zwischen 135 bis 190.

Aus der EP 1745115 B1 ist ein weiteres Verfahren und eine Vorrichtung zum Gewinnen von fraktionierten Kohlenwasserstoffen aus Kunststoffwertstoffen bekannt. In dem hier beschriebenen Verfahren wird zunächst eine verdichtete Masse aus Kunststoffabfällen, insbesondere Polypropylen-, Polyethylen- und Polystyren-Kunststoffen aus Hart- und Weichplastikanteilen in einem Aufschmelzbehälter aufgeheizt, wobei es zu einer Ausbildung einer ersten Flüssigphase und einer ersten Gasphase kommt. Die Flüssigphase und die erste Gasphase werden anschließend in einem Verdampfungsbehälter transportiert, in welchem diese weiter erwärmt werden unter Ausbildung einer zweiten Flüssigphase und einer zweiten Gasphase. Die zweite Flüssigphase wird wiederum in einen Nacherhitzer überführt und dort unter weiterem Wärmeeintrag weiter erhitzt, so dass es zur Ausbildung einer dritten Gasphase kommt. Die zweite Gasphase aus dem Verdampfungsbehälter und die dritte Gasphase aus dem Nacherhitzer werden in einen Crackturm überführt, in welchem ein weiteres Aufbrechen (Cracken) der langkettigen Kohlenwasserstoffe in kurzkettige Kohlenwasserstoffe stattfindet. Die Ausbeute an aus den Kunststoffwertstoffen gebildeten Produktöl liegt zwischen 75 bis 90%, wobei diese abhängig ist von den eingetragenen Kunststoffsorten.

Aus der DE 197 22 586 A1 ist ein weiteres Verfahren bekannt, welches ein Verfahren und Ausrüstungen zur Gewinnung von Paraffinen und/oder Mikrowachsen aus Altkunststoffen beschreibt. Dieses Verfahren dient ausschließlich der Erzeugung von Paraffinen und/oder Mikrowachsen.

Die derzeit bekannten Verfahren zum Abbau von synthetischen Polymeren aus Kunststoffabfall sind jedoch in Bezug auf die Einsetzbarkeit bzw. Verwendbarkeit der hergestellten Produktöle nicht optimal und verbesserungsbedürftig. Eine Verarbeitung von nicht vorsortierten, gereinigten und voragglomerierten Altkunststoffen ist derzeit verfahrenstechnisch in industriellen Anlagen nicht oder nur sehr schwer beherrschbar. Eine Erzielung von klar definierten, reinen und gut verwendbaren Endprodukten aus nicht vorbereiteten Altkunststoffen ist zudem industriell bisher noch nicht gelungen.

Es ist daher eine Aufgabe der vorliegenden Erfindung, ein Verfahren zum Abbau von Kunststoffabfällen mit erhöhter Effizienz und Produktausbeute, insbesondere mit verbesserter Einsetzbarkeit der hergestellten Produktöle, bereitzustellen.

Diese Aufgabe wird mit einem Verfahren mit den Merkmalen des Anspruchs 1 gelöst.

Entsprechend wird ein Verfahren zum Abbau von synthetischen Polyolefinen, wie diese zum Beispiel in Kunststoffabfällen zu finden sind, bereitgestellt, das die folgenden Schritte umfasst:

a) Herstellen einer Schmelze von synthetischen Polyolefinen insbesondere von trockenen synthetischen Polyolefinen

b) Reinigen der Polyolefinschmelze mittels Durchleiten der Polyolefinschmelze durch mindestens einen Schmelzefilter,

c) Überführen der gereinigten Polyolefinschmelze in mindestens einen ersten Reaktor, wobei die gereinigte Polyolefinschmelze in dem mindestens einem ersten Reaktor von einem unteren Bereich in einen oberen Bereich des Reaktors unter Erhitzen auf Temperaturen zwischen 300 °C bis 370 °C, bevorzugt 340 °C bis 360 °C, insbesondere bevorzugt 350 °C, geführt wird, wobei die Pololefine in dem mindestens einem ersten Reaktor in Oligomere gespalten werden,

d) Überführen der in dem mindestens einen ersten Reaktor gebildeten Oligomermischung in mindestens einem zweiten Reaktor, wobei das Oligomergemisch in dem mindestens einen zweiten Reaktor von einem unteren Bereich in einen oberen Bereich des Reaktors unter Erhitzen auf Temperaturen zwischen 380°C bis 450° Celsius, bevorzugt 400 °C bis 430°C, insbesondere bevorzugt 410° Celsius, geführt wird, wobei die Oligomere in dem mindestens einem zweiten Reaktor in Gegenwart von mindestens einem Tonmineral als Depolymersiationskatalysator in kurzkettige Kohlenwasserstoffe abgebaut werden,

e) Abführen der in dem mindestens einen zweiten Reaktor gebildeten kurzkettigen Kohlenwasserstoffen in mindestens einen Vorkondensator, wobei die aus dem mindestens einen zweiten Reaktor austretenden kurzkettigen Kohlenwasserstoffe (C3->C22) in dem mindestens einen Vorkondensator abgekühlt werden; und

f) Einführen der in dem mindestens einen Vorkondensator abgekühlten kurzkettigen Kohlenwasserstoffe in mindestens einen Hauptkondensator, wobei die aus dem mindestens einen Vorkondensator austretenden kurzkettigen Kohlenwasserstoffe in dem mindestens einen Hauptkondensator verflüssigt werden.

Es ist insbesondere bevorzugt, wenn Gemische aus synthetischen Polyolefinen, wie Polyethylen (PE) und Polypropylen (PP), abgebaut werden. Entsprechend ermöglicht das vorliegende Verfahren die Aufarbeitung von nicht-sortenreinen Endverbraucher-Kunststoffabfällen, die insbesondere aus nicht-trennbaren Gemischabfall stammen. Ein besonderer Vorteil des vorliegenden Verfahrens liegt darin, dass das Mischungsverhältnis an Polyethylenen und Polypropylenen vorliegend unwichtig ist. So können alle beliebigen Mischungsverhältnisse aus allen Polyethylensorten und allen Polypropylensorten verwendet werden; eine aufwendige Trennung der Polyolefinabfälle ist nicht notwendig.

Ein weiterer Vorteil des vorliegenden Verfahrens besteht darin, dass aufgrund des Abbaus der Polyolefine zu kurzkettige Kohlenwasserstoffen mit einer definierten Zusammensetzung Energieträger in Form von Heizölen und Benzinfraktionen gebildet werden und gleichzeitig Plastikabfälle beseitigt werden.

Zudem wird durch die Herstellung von definierten Heizöl- und Benzinfraktionen Energie gespeichert, die bei Bedarf abrufbar ist, zum Beispiel durch Verbrennen in Standheizungen oder Blockheizkraftwerken. Im Gegensatz dazu werden zwar bei der zum Großteil eingesetzten Müllverbrennung die Plastikabfälle beseitigt, jedoch ist dieses Verfahren kostenintensiv und generiert lediglich als Nebenprodukt Energie.

Das vorliegende Verfahren bewirkt die Erzeugung von synthetischen Ölen (Olefinen) und Benzin und damit verkaufsfähigen Produkten, die im Bereich des Benzin-Kraftstoffs und des Heizöls liegen. Wie beschrieben werden die synthetischen Öl- und Benzinprodukte aus Kunststoffabfallfraktionen des Typs Polyolefine (PE/PP) durch die chemische Reaktion einer schonend verlaufenden Niedrigtemperaturpyrolyse bzw. Thermolyse unter Verwendung einer optimierten Verweilzeitsteuerung, einer optimierten Temperatursteuerung und einer optimierten Steuerung der Reaktorkopftemperatur gezielt erzeugt.

Die zum Einsatz kommenden synthetischen Polymere bzw. Inputstoffe, bestehen zu über 95% aus Polyethylen und Polypropylengemischen. Diese Plastikabfälle werden in einer Ausführungsform des vorliegenden Verfahrens zunächst zerkleinert und dann einer Metall-, Nichtmetall- und Schwerstoffabscheidung unterzogen. In weiteren Schritten erfolgt eine Abtrennung von chlorierten Kunststoffen (Chlorentfrachtung), Eliminierung von gegebenenfalls enthaltener Zellulose, Waschen, Abpressen, Trocknen und Verdichtung. Die durch die Aufarbeitung anfallenden Abfallstoffe wie Mineralien, Metalle, Faserstoffe, Papier und Plastik werden entsprechend entsorgt bzw. biogene Abfallstoffe können in mit dem Waschwasser ausgetragen werden.

Die vorstehend beschriebenen, aufbereiteten Altkunststoffe liegen nunmehr als lose und gereinigte Kunststoffschnitzel vor, die einer Voragglomerisierung unterzogen werden und damit eine Konsistenz erlangen, welche den Eintrag in das vorliegende Verfahren ermöglicht.

In einer Ausführungsform des vorliegenden Verfahrens werden die aufgearbeiteten, voragglomerisierten Polyolefine in einem Extruder, insbesondere in einen Doppelschneckenextruder, bei Temperaturen bis zu 300 °C aufgeschmolzen. Die aufbereiteten Polyolefine werden unter Verwendung einer Dosiereinheit als Zuführeinrichtung kontinuierlich in den Extruder eingeführt, wobei die verwendete Dosiereinheit eine massenkontrollierte Zufuhr des Polyolefins in den Extruder ermöglicht. In der Dosiereinrichtung erfolgt gleichzeitig eine weitere Trocknung und Vorwärmung der Polyolefine Die Regelung der Zufuhr der aufbereiteten Polyolefine in den Extruder erfolgt dabei in Abhängigkeit des Polymerabbaus in dem mindestens ersten und mindestens zweiten Reaktor, zum Beispiel unter Verwendung einer Füllstandsmessung (z. B. in Form einer Wägeeinrichtung).

Durch die im Extruder erfolgende Erwärmung und Aufschmelzung der Polymere kommt es gleichzeitig zur Entfernung einer gegebenenfalls in den Polyolefinen enthaltenen Restfeuchte sowie zu einer Entgasung.

In einer weiteren Variante des vorliegenden Verfahrens beträgt die Viskosität der Polyolefinschmelze am Extruderausgang $1{,}5 \times 10^6$ bis $2 \times 10^6$ mPas (300°C), bevorzugt $1{,}5 \times 10^6$ mPas (300°C).

Die Viskositäten der Polymerschmelze wurden über die Messungen der Schmelzfließraten mittels eines Rheometers unter Berücksichtigung der thermischen Degradation bei den entsprechenden Temperaturen ermittelt. Generell besteht ein Zusammenhang zwischen der Schmelzfließrate (MVR) und der Schmelzeviskosität $\eta^*$.

Die Schmelzviskosität $\eta^*$ ergibt sich gemäß

$$\eta^* = \frac{\tau_S}{\dot{\gamma}_S}$$

Wobei $\tau_S$ für die Schubspannung steht und $\gamma_S$ für die Schergeschwindigkeit steht, Die Schubspannung $\tau_S$ berechnet sich gemäß

$$\tau_S = \frac{r_D}{2 \cdot l_D} \cdot \frac{m \cdot g}{\pi \cdot r_Z^2}$$

mit r= Radius, d = Durchmesser, l = Länge, m = Last, g = Erdbeschleunigung.

Die Schergeschwindigkeit ergibt sich aus

$$\dot{\gamma}_S = \frac{4 \cdot MVR}{\pi \cdot r_D^2}$$

mit MVR = Schmelzfließrate.

Die Geometrie des verwendeten Prüfgeräts ist Z: Zylinder (dZ= 9.550 mm), D: Düse (dD= 2.095 mm, ID= 8.0 mm).

Die aus dem Extruder austretende Polymerschmelze wird anschließend durch eine beheizte Leitung bevorzugt in eine erste Schmelzepumpe gefördert. In der Folge wird die Polymerschmelze durch den mindestens einen Schmelzefilter geführt, in welchen bevorzugt restlichen Spuren von Verunreinigungen, insbesondere von Mineralien, Metallen, Faserstoffen, aus der Polymerschmelze entfernt werden. Bevorzugterweise ist der mindestens eine Schmelzefilter zwischen dem mindestens einem Ausgang des Extruders und mindestens einer Eintrittsöffnung in den mindestens einen ersten Reaktor angeordnet. Mit anderen Worten, der Schmelzefiliter kann zwischen Extruderausgang und einem beheiztem Rohr, das in den mindestens einen ersten Reaktor mündet, vorgesehen sein. Der mindestens eine Schmelzefilter verwendet bevorzugt ein Filtermaterial in Form eines Cr/Ni-Stahlnetzes mit einer Porengröße (Maschenweite) zwischen 100 bis 400 μm, die je nach Teilchengröße der aus der Polymerschmelze zu entfernenden Verunreinigungen gewählt wird. Generell wäre auch die Verwendung von Filterplatten (Siebplatten) oder Filterkerzen z.B. aus Edelstahl als Filtermaterial möglich.

Die Kontrolle bzw. Steuerung der Filterfunktion des Schmelzefilters erfolgt bevorzugt mittels eines vor dem Schmelzefilter angeordneten Drucksensors, der wiederum mit einer zentralen Anlagensteuerung verbunden sein kann.

In einer weiteren Ausführungsform des vorliegenden Verfahrens wird die aus dem Schmelzefilter austretende Polymerschmelze mittels einer geeigneten Schmelzpumpe in den unteren Bereich des mindestens ersten Reaktors eingeführt, wobei die Polymerschmelze bei Eintritt in den ersten Reaktor, und hier insbesondere in den unteren Bereich des ersten Reaktors, eine Viskosität von 1,5 x 10$^6$ bis 2 x 10$^6$ mPas (300°C), bevorzugt 1,5 x 10$^6$ mPas (300°C) aufweist.

Die im vorliegenden Verfahren zum Einsatz kommenden Schmelzepumpen sind bevorzugterweise temperaturgeführte Zahnradpumpen, die beheizbar sind und auf dem jeweils eingestellten Temperaturniveau haltbar sind. Auch weisen die Schmelzepumpen bevorzugt eine Rücklaufsperre auf, um so den Rücklauf der Polymerschmelze in der Anlage zu verhindern. Die Schmelzepumpen bestehen bevorzugterweise aus Edelstahl und werden bevorzugt durch die Polymerschmelze geschmiert.

Der mindestens eine erste Reaktor, der zum Beispiel in Form eines Rührkesselreaktors vorliegen kann, umfasst mehrere Temperaturbereiche, insbesondere mehrere axiale Heizzonen mit mindestens einem Rührorgan. In dem mindestens einen ersten Reaktor erfolgt eine kontrollierte und schonende Vorspaltung und Konditionierung der Polymere durch Kontrolle der Reaktionsparameter Temperatur, Druck, Verweilzeit und Viskosität. Zudem steht der erste Reaktor mit einer Wägeeinrichtung in Verbindung (ist z.B. auf einer Wägeeinrichtung bzw. Wägezelle platziert), so dass zu jedem Zeitpunkt der aktuelle Füllstand des ersten Reaktors an die Anlagensteuerung gemeldet werden kann. Dies ermöglicht eine kontinuierliche Fahrweise der gesamten Anlage und somit ein kontinuierliches Verfahren.

Das in dem mindestens einen ersten Reaktor verwendete Rührorgan kann in verschiedenen Ausführungsformen vorliegen. So ist es möglich, ein Rührorgan in Form eines Wendelrührers, Ankerrührers, Schnecke oder eine Kombination davon einzusetzen. Bevorzugt wird jedoch ein Rührorgan verwendet, das eine Kombination aus Wendelrührer und Schnecke, das heißt eine Rührwelle mit Wendelrührer und Schnecke, darstellt.

Der zum Einsatz kommende Rührer ist auf das Verfahren abgestimmt. So ist der Antrieb an die an die jeweiligen Viskositäten angepasst. Die Werkstoffeigenschaften sind so gewählt, dass der Rührer für Polyolefinschmelzen und die entsprechenden Temperaturbereiche geeignet ist. Die Dimensionen des Rührers sind in Anpassung an die Reaktoren gewählt. Die bevorzugt verwendete Rührblattkombination aus Wendel und Schnecke ermöglicht die Erzeugung von Auftrieb entlang der Reaktorwandung und Abtrieb im Reaktorzentrum. Der Rührer begünstigt den Eintrag und die Verteilung der zugeführten Energie und beschleunigt die Umsetzraten signifikant.

Der mindestens eine erste Reaktor weist bevorzugt ein Verhältnis von Höhe:Durchmesser (H:D) von 7:1, bevorzugt 5,5:1 auf. Die gewählten Reaktordimensionen ermöglichen einen effizienten Energieeintrag, die Vermeidung von großen Temperaturabweichungen bzw. - schwankungen und ein flaches Temperaturprofil durch den gesamten Reaktor.

Bevorzugterweise tritt die gereinigte Polymerschmelze in einen unteren Bereich (Sumpfzone) des ersten Reaktors mit einer Temperatur von 220°C bis 300°C, bevorzugt 300°C ein und wird beim Aufsteigen im ersten Reaktor in einem Temperaturgradienten auf Temperaturen zwischen 330°c und 360 °C, bevorzugt 350°C am Reaktorkopf des ersten Reaktors erwärmt.

In dem mindestens einen ersten Reaktor erfolgt bevorzugt eine Vorspaltung bzw. ein Abbau der Polymere mit einem typischen Molekulargewicht von > 10$^5$ kg/Mol in Oligomere mit einem Molekulargewicht zwischen 10$^2$ bis 10$^4$ kg/Mol, bevorzugt 10$^3$ bis 10$^4$ kg/Mol.

In einer Variante kann das den oberen Bereich (Kopfzone) des ersten Reaktors verlassene Oligomergemisch eine Viskosität zwischen 500 und 1500 mPas (350°C), bevorzugt 500 und 1.000 mPas (350°C), insbesondere bevorzugt zwischen 600 und 800 mPas (350°C) aufweisen. Ein typischer Viskositätswert des Oligomergemisches am Reaktorkopf des ersten Reaktors beträgt 650 bis 700 mPas (350°C). Die während der stufenweisen Erwärmung des im ersten Reaktor befindlichen Polymers erfolgende Viskositätsabnahme generiert einen Auftrieb des sich bildenden Produktgemisches (Oligomergemisches) entlang der Wandung des ersten Reaktors und einen gleichzeitigen Abtrieb im Kern (sogenannte Schlaufenströmung oder laminare Schmelzeströmung). Dieser Effekt kann durch das Rührorgan verstärkt werden.

Wie bereits erwähnt, wird die in dem mindestens einen ersten Reaktor ablaufende Vorspaltung durch die Reaktionsparameter Temperatur, Druck, Verweilzeit und Viskosität kontrolliert. So beträgt die Temperatur im oberen Bereich des mindestens einen ersten Reaktors 340°C bis 360 °C, bevorzugt maximal 350°C. Die kontrollierte und schonende Vorspaltung und Konditionierung der Polymere zu Oligomeren in dem mindestens einen ersten Reaktor vermeidet instabile Betriebszustände in der weiteren Spaltung in dem folgenden zweiten Reaktor. Ebenfalls verhindert ein schonendes, graduelles Erwärmen der Polymerschmelze im ersten Reaktor ein Aufschäumen der Polymerschmelze.

Während der Vorspaltung der Polymere in dem mindestens einen ersten Reaktor in die entsprechenden Oligomere kann es zur Bildung von geringen Mengen an gasförmigen Kohlenwasserstoffen kommen, die in einer Ausführungsform über eine Fackel abgeführt und somit verbrannt werden oder in einer anderen Ausführungsform zur Energieerzeugung zum Beispiel in einer Gasturbine verwendet werden können. Die im ersten Reaktor anfallenden gasförmigen Depolymerisationsprodukte können auch über eine geheizte Leitung in einen noch weiter unten beschriebenen Vorkondensator eingeleitet werden.

In einer weiteren bevorzugten Variante des vorliegenden Verfahrens wird die in dem mindestens einen ersten Reaktor gebildete Oligomerschmelze am oberen Bereich, z.B. Reaktorkopf, des ersten Reaktors abgeführt und mittels einer Schmelzpumpe über eine Rohrleitung, bevorzugt über eine beheizte Rohrleitung, kontrolliert in den mindestens einen zweiten Reaktor, insbesondere in den unteren Bereich (Sumpfzone) des zweiten Reaktors eingeführt.

In einer bevorzugten Ausführungsform des vorliegenden Verfahrens wird das den mindestens einen ersten Reaktor verlassene Oligomergemisch vor Einführen in den mindestens einen zweiten Reaktor mit dem mindestens einen Tonmineral als Depolymerisationskatalysator vermischt. Hierzu wird in die aus dem ersten Reaktor austretende Oligomerschmelze das mindestens eine Tonmineral über eine in die Rohrleitung zwischen ersten und zweiten Reaktor einmündende Leitung eingeführt. Die das Tonmineral einführende Leitung weist bevorzugt einen Stutzen, bevorzugt in Form eines sog. "Hosenrohrs" auf. Die das Tonmineral einführende Leitung endet mit dem genannten Stutzen bevorzugt in der Mitte bzw. Kernzone der Rohrleitung zwischen ersten und zweiten Reaktor. Wie später noch im Detail erläutert wird das mindestens eine Tonmineral in einem in einem separaten Mischbehälter hergestellten Gemisch aus Tonmineral, Paraffinen und Mikrowachsen in die den ersten Reaktor verlassene Oligomerschmelze eingeleitet. Bevorzugt werden 2-3% Tonmineral pro Tonne Kunststoff verwendet.

In einer Variante des vorliegenden Verfahrens ist das mindestens eine Tonmineral ausgewählt aus der Gruppe enthaltend Schichtsilikate, insbesondere Montmorrillonit, IIlit, Bentonit, Kaolinit, Smektit, Chlorit, Vermikulit und Glimmer. Es können auch sog. Wechsellagerungsminerale wie z.B. Kaolinit/Smektit, Chlorit/Vermikulit, Glimmer/Vermikulit oder sehr häufig Wechsellagerung von IIlit/Smektit oder IIlit/Montmorillonit zum Einsatz kommen. Bevorzugte Tonminerale sind Bentonit und Montmorillonit.

Es ist ebenfalls möglich, ein vorbehandeltes Tonmineral zu verwenden. Die Vorbehandlung kann mit einer Säure, insbesondere einer anorganischen Säure wie Schwefelsäure, Salpetersäure oder Phosphorsäure erfolgen, wobei Schwefelsäure besonders bevorzugt ist.

Der mindestens eine zweite Reaktor weist (wie auch der erste Reaktor) bevorzugt ein Verhältnis von Höhe:Durchmesser (H:D) von 7:1, bevorzugt 5,5:1 auf.

Bevorzugterweise weist die in den mindestens einen zweiten Reaktor eingeführte Oligomerschmelze eine Temperatur zwischen 300°C und 370 °C, bevorzugt von 350° Celsius auf. Die Oligomerschmelze wird in dem mindestens einen zweiten Reaktor schonend in einen Temperaturgradienten mit Temperaturen zwischen 380°C und 450 °C, bevorzugt 410 °C von unten nach oben geführt. Entsprechend liegt die Temperatur im Reaktorkopf bevorzugt in einem Bereich zwischen 380°C und 450°C, bevorzugt 400 °C und 410 °C.

Es ist bevorzugt, dass in dem mindestens einen zweiten Reaktor die Spaltung der Oligomere in kurzkettige Kohlenwasserstoffe mit einem Molekulargewicht von <500 kg/Mol erfolgt. Dabei erfolgt die Aufspaltung der Oligomere in kurzkettige, unter den Reaktionsbedingungen des zweiten Reaktors gasförmig vorliegende Kohlenwasserstoffe bevorzugt im oberen Bereich des mindestens einen zweiten Reaktors, d.h. im Reaktorkopf desselbigen. Typische kurzkettige Kohlenwasserstoffe, die im zweiten Reaktor gebildet werden, sind C3->C22, bevorzugt C3-C25-Kohlenwasserstoffe, insbesondere ungesättigte Kohlenwasserstoffe, die in verschiedenen Fraktionen anfallen, wie später noch detaillierter erläutert wird.

Die in den mindestens einen zweiten Reaktor aus dem mindestens einen ersten Reaktor eintretende Oligomerschmelze kann eine Viskosität zwischen 500 und 1.500 mPas (350°C), bevorzugt zwischen 500 und 1.000 mPas (350°C), insbesondere bevorzugt zwischen 600 und 800 mPas (350°C), ganz insbesondere bevorzugt zwischen 650 und 700 mPas (350°C) aufweisen.

Die am oberen Ende des mindestens einen zweiten Reaktors, das heißt im Reaktorkopf desselbigen, gebildete Kohlenwasserstofffraktion kann eine Viskosität zwischen 50 und 300 mPas (410°C), bevorzugt zwischen 100 und 250 mPas (410°C), insbesondere bevorzugt zwischen 150 und 200 mPas (410°C) aufweisen.

Aufgrund der abnehmenden Viskosität des sich in dem mindestens einen zweiten Reaktor befindlichen Oligomergemisches wird - wie bereits für den ersten Reaktor beschrieben - entlang der Wandung des zweiten Reaktors ein Auftrieb des Oligomergemisches und gleichzeitig ein Abtrieb desselbigen im Reaktorkern erzeugt (sogenannte Schlaufenströmung), wobei dieser Effekt durch einen Rührer verstärkt wird. Die in dem mindestens einen zweiten Reaktor verwendeten Rührorgane entsprechen bevorzugt den Rührorganen für den mindestens einen ersten Reaktor, ebenso wie das Verhältnis von Höhe zu Durchmesser.

Die Temperaturen in dem mindestens einen ersten Reaktor und dem mindestens einen zweiten Reaktor werden bevorzugterweise an die Zusammensetzung des jeweils verwendeten Polymerausgangsgemisches angepasst, wobei die Temperaturdifferenz zwischen dem zweiten Reaktor und ersten Reaktor zwischen 20 bis 100°C, bevorzugt zwischen 30 und 70 °C, insbesondere bevorzugt zwischen 40 und 60 °C betragen kann.

Es ist ebenfalls möglich und denkbar, dass nicht nur mindestens ein erster und mindestens ein zweiter Reaktor verwendet werden, sondern dass mehr als zwei, z.B. drei oder vier parallel zueinander angeordnete Reaktoren zum Einsatz kommen.

In einer weiteren Variante des vorliegenden Verfahrens werden die im Reaktorkopf des mindestens einen zweiten Reaktors gebildeten kurzkettigen gasförmigen Kohlenwasserstoffe (z.B. C3 bis >C22), bevorzugt schnell, über eine Rohrleitung, bevorzugt beheizte Rohrleitung, in mindestens einen Vorkondensator abgeführt, und die in dem Vorkondensator abgekühlten gasförmigen Kohlenwasserstoffe werden zur weiteren Abkühlung und Kondensation in den mindestens einen Hauptkondensator eingeleitet. Der Vorkondensator kann in Form eines Spiralrohrwärmeübertragers ausgebildet sein.

In dem mindestens einen Vorkondensator erfolgt bevorzugterweise eine Kühlung der gasförmigen Kohlenwasserstoffe auf Temperaturen zwischen 300 und 400 °C, bevorzugt 330 und 370 °C, insbesondere bevorzugt auf 350 °C, um weitere ungewollte Folgereaktionen, wie zum Beispiel weitere Degradationsreaktionen, zu unterdrücken. Mit der spontanen Abkühlung der gasförmigen Depolymerisationsprodukte im Vorkondensator auf 350 °C, wird der Depolymerisationsprozess somit beendet und somit eine signifikante Verschiebung der Produktskala zum Flüssiggasanteil verhindert.

Aufgrund der Abkühlung der gasförmigen Kohlenwasserstoffe im Vorkondensator kann es zur Abscheidung von Paraffinen (d.h. von mehrheitlich höheren gesättigten Kohlenwasserstoffen mit einer Kettenlänge über C22) kommen, die bevorzugt in flüssiger oder pastöser Form über eine ebenfalls beheizte Rohrleitung in mindestens einen Mischbehälter abgeführt werden.

In dem mindestens einen Mischbehälter (bevorzugt als Rührmaschine ausgelegt) werden die im Vorkondensator abgeschiedenen flüssigen Paraffine bevorzugterweise mit dem Edukt (hier Oligomergemisch) aus dem unteren Bereich des mindestens einen zweiten Reaktors vermengt bzw. vermischt. Die Vermischung von Paraffinen und Oligomeren im Mischbehälter erfolgt bevorzugt unter Verwendung eines geeigneten Rührwerks, wie zum Beispiel eines Wendelrührers. Zusätzlich wird in den Mischbehälter das mindestens eine Tonmineral eingeführt. Das Tonmineral z.B. Bentonit wird pulverförmig in das Gemisch aus Paraffinen, Oligomeren bzw. Mikrowachsen eingerührt. Wie oben bereits erläutert, wird dieses pastöse Gemisch aus Tonmineral, Paraffinen und Oligomeren über eine beheizte Rohrleitung und eine Schmelzepumpe in die Rohrleitung zwischen ersten und zweiten Reaktor unter Verwendung eines hosenförmigen Spezialstutzens eingeführt.

Die Temperatur des Gemisches im Mischbehälter wird bevorzugt bei Temperaturen zwischen 300 °C und 400 °C, bevorzugt 330 °C und 370 °C, insbesondere bevorzugt bei 350 °C gehalten.

Es ist aber auch möglich, dass die Mischung aus Oligomeren und Paraffinen (ohne Tonmineral), die bevorzugt eine Temperatur von 350 °C aufweist, zusätzlich in den mindestens einen zweiten Reaktor unter Verwendung einer Schmelzepumpe in die Reaktionszone, d.h. bevorzugt in den unteren Bereich/Sumpfzone oder auch in den mittleren bis oberen Bereich des mindestens einen zweiten Reaktors, rückgeführt und erneut dem Spaltprozess unterworfen wird. Die Oligomer-Paraffin-Mischung wird in dem mindestens einen zweiten Reaktor bevorzugt als Dispergiermittel eingesetzt.

In einer Variante des folgenden Verfahrens erfolgt die Kondensation der aus dem mindestens einen zweiten Reaktors via Vorkondensator in den Hauptkondensator eingeleiteten gasförmigen Kohlenwasserstoffe bei Temperaturen zwischen 15 und 30°C, bevorzugt bei 20°C, wobei es zur fast vollständigen Verflüssigung der gasförmigen Kohlenwasserstoffe kommt. Nicht kondensierbare Gase können entweder verbrannt werden, z.B. durch Ableiten der Gase aus dem Hauptkondensator und Einleiten durch eine Gasuhr in eine Fackel oder können zur weiteren energetischen Nutzung für die Anlage oder andere Zwecke z.B. mittels einer Gasturbine eingesetzt werden.

Der Hauptkondensator ist bevorzugt als schrägliegender Spiralrohrwärmeübertrager ausgeführt. Die Schräglage von 10- 30°, bevorzugt 20° des Hauptkondensators ist für die optimale Trennung der gasförmigen und flüssigen Phase und deren Ableitung nach unten gewählt. Die Temperaturen im Kopf des Hauptkondensators werden bevorzugt mit 20 °C bis 25 °C eingestellt. Damit werden Restgase und flüchtige Bestandteile der Depolymerisationsprodukte der Kettenlängen C3 bis C6 als gasförmige Bestandteile oben aus dem Hauptkondensator austreten. Dieses Abgas wird - wie oben angeführt - über eine geheizte Rohrleitung zur Fackelanlage geführt und dort verbrannt. Das aus dem Hauptkondensator nach unten in einen Produktsammelbehälter abgeleitete Produkt ist ein synthetisches Produktöl und besteht aus gesättigten und ungesättigten Kohlenwasserstoffen in einem Siedebereich von 40 °C bis 350 °C, bevorzugt 60 und 350°C.

So umfasst das hergestellte Produktöl die Kohlenwasserstofffraktionen Flüssiggas (C3-C6), Benzin (C7-C10), Kerosin (C11-C13), Gasöl (C14-C19), schweres Gasöl (C20-C22) und Paraffine/Mikrowachse (>C22).

Der quantitative Anteil der Fraktionen unterscheidet sich in Abhängigkeit von dem verwendeten Abfallpolymer und der in dem mindestens einen zweiten Reaktor angewendeten Thermolysetemperatur.

Generell ist zu sagen, dass bei niedrigeren Thermolysetemperaturen von z.B. 385°C oder 400°C der Anteil an niederen Kohlenwasserstoffen (C3-C13) größer ist als bei höheren Temperaturen z.B. von 415°C, bei welcher der Anteil an höheren Kohlenwasserstoffen (>C14) liegt, wobei die Zusammensetzung des Ausgangspolymergemisches eine Einfluss auf die quantitativen Anteile hat.

Das vorliegende Verfahren ermöglicht demnach durch die Wahl der Thermolysetemperatur im zweiten Reaktor, die Produktverteilung hin zu einer gewünschten Produktgruppe zu lenken.

Das aus Polypropylen (PP) gewonnene Kondensat umfasst bevorzugt oligomere Einheiten von Propen, die beim Abbau des PP durch Spaltung der C-C-Bindungen entstehen. Das PP-Kondensat aus dem Hauptkondensator kann typischerweise folgende Fraktionen umfassen: Flüssiggas C3-C6, Benzin C7-C10, Kerosin C11-C13, Gasöl C14-C19, schweres Gasöl C20-C22 und Paraffine/Mikrowachse > C22. Die Produkte erstrecken sich dabei über einen weiten Kettenlängenbereich von C3-C30. So wird z.B. Polypropylen (PP) als Startpolymer bevorzugt in C3-Monomere (C3), C3-Dimere (C6) C3-Trimere (C8-C10), C3-Pentamere (C14-C16), C3-Heptamere (C20-C22) und C3-Nonamere (C26-C28) aufgespalten. Hauptspaltprodukt ist 2,4-Dimethyl-Hept-1-en (C9).

Wie bereits erwähnt, wird die quantitative Zusammensetzung des Produktöles durch die Ausgangspolymere sowie durch die Thermolysetemperaturen im zweiten Reaktor bestimmt.

So weist ein bei einer Thermolysetemperatur von 385°C gewonnenes PP-Produktöl z.B. folgende Zusammensetzung auf: ca. 8-12 Gew% Paraffine/Mikrowachse > C22; ca. 8-12 Gew% schweres Gasöl C20-C22; ca. 22-27 Gew% Gasöl C14-C19; ca. 12-17 Gew% Kerosin C11-C13; ca. 27-32 Gew% Benzin C7-C10 und ca. 8-12 Gew% Flüssiggas C3-C6.

Ein bei einer Thermolysetemperatur von 400°C gewonnenes PP-Produktöl weist z.B. folgende Zusammensetzung auf: ca. 22-27 Gew% Paraffine/Mikrowachse > C22; ca. 8-12 Gew% schweres Gasöl C20-C22; ca. 18-22 Gew% Gasöl C14-C19; ca. 12-17 Gew% Kerosin C11-C13; ca. 22-27 Gew% Benzin C7-C10 und ca. 3-7 Gew% Flüssiggas C3-C6.

Ein bei einer Thermolysetemperatur von 415°C gewonnenes PP-Produktöl weist z.B. folgende Zusammensetzung auf: ca. 28-32 Gew% Paraffine/Mikrowachse > C22; ca. 8-12 Gew% schweres Gasöl C20-C22; ca. 22-27Gew% Gasöl C14-C19; ca. 8-12 Gew% Kerosin C11-C13; ca. 18-22 Gew% Benzin C7-C10 und weniger als ca. 5 Gew% Flüssiggas C3-C6.

Ein aus Polyethylen (PE) gewonnenes Kondensat umfasst bevorzugt Kohlenwasserstoffe in einem Kettenlängenbereich von C3-C30. Das PE-Kondensat umfasst typischerweise n-Alkane und n-Alkene (Olefine) in einem Verhältnis von 50:50. So liegen die Kohlenwasserstoffe parallel immer in der gesättigten und ungesättigten Form vor wie z.B. C10: n-Undecen und Undec-1-en.

Ein bei einer Thermolysetemperatur von 400°C gewonnenes PE-Produktöl weist z.B. folgende Zusammensetzung auf: Spuren von Paraffine/Mikrowachse > C22; Spuren von schwerem Gasöl C20-C22; ca. 28-32 Gew% Gasöl C14-C19; ca. 18-22 Gew% Kerosin C11-C13; ca. 42-47 Gew% Benzin C7-C10 und ca. 5 -10 Gew% Flüssiggas C3-C6.

Ein bei einer Thermolysetemperatur von 410°C gewonnenes PE-Produktöl weist z.B. folgende Zusammensetzung auf: Spuren von Paraffine/Mikrowachse > C22; weniger als 5 Gew% schweres Gasöl C20-C22; ca. 42-47 Gew% Gasöl C14-C19; ca. 12 -17 Gew% Kerosin C11-C13; ca. 28-32 Gew% Benzin C7-C10 und ca. 5 -10 Gew% Flüssiggas C3-C6.

Ein bei einer Thermolysetemperatur von 415°C gewonnenes PE-Produktöl weist z.B. folgende Zusammensetzung auf: Spuren von Paraffine/Mikrowachse > C22; weniger als 5 Gew% schweres Gasöl C20-C22; ca. 42-47 Gew% Gasöl C14-C19; ca. 12-17 Gew% Kerosin C11-C13; ca. 28-32 Gew% Benzin C7-C10 und ca. 5 -10 Gew% Flüssiggas C3-C6.

Ein bei einer Thermolysetemperatur von 420°C gewonnenes PE-Produktöl weist z.B. folgende Zusammensetzung auf: ca. 3-7 Gew% Paraffine/Mikrowachse > C22; ca. 8-12 Gew% schweres Gasöl C20-C22; ca. 42-47 Gew% Gasöl C14-C19; ca. 12-17 Gew% Kerosin C11-C13; ca. 22-27 Gew% Benzin C7-C10 und ca. 5 -10 Gew% Flüssiggas C3-C6.

In einer weiteren bevorzugten Ausführungsform wird das den Hauptkondensator verlassene Kondensat bzw. Produktöl einer Destillation zur Auftrennung des Kondensats in Fraktionen mit unterschiedlichen Siedepunkten zugeführt. In diesem Fall entfällt der Hauptkondensator und wird durch eine Destillationskolonne (fraktionierte Destillation) ersetzt. Hier kommt es zur Auftrennung der Fraktionen "Heizöl" und Benzin. Fraktionskolonnen sind gängige Technologien und werden vielfältig verwendet. Die Dimensionierung der Fraktionskolonnen wird bevorzugt an die Siedebereiche der einzelnen Fraktionen angepasst, wobei Kolonnenhöhe, Volumen, Bodenzahl und/oder Kopfkondensator der Kolonne veränderbar sind.

Das den Hauptkondensator verlassende Kondensat bzw. Produktöl kann in einem Auffangbehälter gesammelt werden und anschließend bevorzugt einer Destillation bzw. Rektifikation zugeführt werden, die eine Auftrennung des Produktöls in eine benzinähnliche Fraktion und eine heizölähnliche Fraktion bewirkt.

Die Aufgabe der vorliegenden Erfindung wird ebenfalls mit einer Anlage zur Durchführung des vorliegenden Verfahrens gemäß Anspruch 16 gelöst.

Entsprechend umfasst eine Anlage zur Durchführung des beschriebenen Verfahrens

- mindestens einen Extruder zum Schmelzen der synthetischen Polymere,
- mindestens einen stromabwärts vom Extruder angeordneten Schmelzefilter zur Reinigung der Polyolefinschmelze
- mindestens einen stromabwärts vom Schmelzefilter angeordneten ersten Reaktor zum Abbau der Polyolefine in Oligomere,
- mindestens einen stromabwärts von dem mindestens einen ersten Reaktor angeordneten zweiten Reaktor zum Abbau der in dem mindestens einen ersten Reaktor gebildeten Oligomere in gasförmige Kohlenwasserstoffe,
- mindestens einen stromabwärts von dem mindestens einen zweiten Reaktor angeordneten Vorkondensator zum Vorkühlen der den zweiten Reaktor verlassenen kurzkettigen gasförmigen Kohlenwasserstoffe, und
- mindestens einen stromabwärts von dem mindestens einen Vorkondensator angeordneten Hauptkondensator zur Kondensation der in dem mindestens einen Vorkondensator vorgekühlten gasförmigen Kohlenwasserstoffe.

Die für das vorliegende Verfahren beschriebenen Reaktionsbedingungen und die damit verbundenen Ausbildungen der einzelnen Teile bzw. Komponenten der folgenden Anlage sind entsprechend anwendbar, so dass auf eine Wiederholung im Folgenden darauf verwiesen wird.

In einer Variante der folgenden Anlage verfügen der verwendete mindestens eine erste Reaktor und der mindestens eine zweite Reaktor jeweils über mindestens zwei regelbare Heizzonen, bevorzugt über mindestens drei bis fünf regelbare Heizzonen. Die Heizzonen werden durch in der Wandung der Reaktoren angeordnete Heizungen eingestellt. In jeder Heizzone sind Temperatursensoren vorgesehen, die die jeweils aktuellen Temperaturen in der entsprechenden Heizzone der Anlagensteuerung übersenden. Die Anzahl der Heizzonen kann temperaturmäßig beliebig eingestellt werden und ist insbesondere abhängig von der Zusammensetzung der abzubauenden Polyolefinschmelze Auch kann die Anzahl der Heizzonen in dem mindestens einen ersten Reaktor gleich oder unterschiedlich der Anzahl der Heizzonen in dem mindestens einen zweiten Reaktor sein. Durch die Heizzonen ist die Einstellung eines Temperaturgradienten in den Reaktoren möglich, wobei die Temperatur innerhalb der Reaktoren von unten nach oben ansteigt.

Des Weiteren weisen der mindestens eine erste Reaktor und der mindestens eine zweite Reaktor jeweils ein Rührorgan in Form eines Wendelrühres, eines Ankerrührers, einer Schnecke oder eine Kombination davon auf, wobei beide Reaktoren bevorzugt über ein Rührorgan in Form einer Kombination aus Wendelrührer und Schnecke verfügen.

In einer bevorzugten Ausführungsform weisen die Innenwände und Einbauten des mindestens einen ersten Reaktors und/oder des mindestens einen zweiten Reaktors mindestens eine keramische Beschichtung auf. Eine derartige keramische Beschichtung hat sich als vorteilhaft erwiesen, da hierdurch der Prozess des Verkokens stark reduziert werden kann. Reaktoren aus Edelstahl zeigen unter gleichen Prozessbedingungen, wenn auch mittels des Einsatzes der speziellen Rührorgane verringert, eine Kohlenstoffbildung an den Innenwänden der Reaktoren. Es wurde zudem gefunden, dass Stahllegierungen einen unerwünschten katalytischen Zerfallsprozess an den kurzkettigen Kohlenwasserstoffmolekülen, C3-C22-Kohlenwasserstoffe, zu Kohlenstoff und Wasserstoff begünstigen. Zur Vermeidung dieses Effektes kann eine komplette Inertisierung mittels keramischer Innenbeschichtung der betroffenen Anlagenbauteile vorgesehen werden. Die zu beschichteten Bauteile sind neben den Innenwänden des ersten und/oder zweiten Reaktors, die Oberfläche der jeweils in den Reaktoren verwendeten Rührorgane, die Rohrleitung zwischen ersten und zweiten Reaktor, die Rohrleitung zwischen zweitem Reaktor und Vorkondensator, die Innenwände des Vorkondensators und die Kühlelemente im Vorkondensator. Die keramische Beschichtung weist bevorzugt eine Dicke zwischen 1 und 5 mm, bevorzugt 1 und 2 mm Stärke auf. Die keramische Beschichtung ist chemisch inert, temperaturbeständig bis 550°C sowie temperaturdurchlässig. Kontakte in den kritischen Temperaturbereichen zwischen der Polymerschmelze und den Crack - Gasen mit der Edelstahloberfläche sind damit ausgeschlossen. Die keramische Beschichtung zeichnet sich zudem durch eine lange Haltbarkeit aus, widersteht mechanischen Beanspruchungen (kratz- und schlagfest) und wirkt Schmutzablagerungen entgegen. Dies bewirkt zusätzlich eine Verlängerung der Zeiträume zwischen den Wartungsintervallen und eine Vereinfachung der Reinigung der beschichteten Bauteile im Rahmen einer Wartung. Bevorzugte Keramikbeschichtungen basieren auf Siliziumkarbiden. So ist z.B. Si dotiertes Siliziumkarbid durch eine hohe Härte, Wärmeleitfähigkeit, chemische Beständigkeit und Korrosionsfestigkeit charaktersiert.

Die Reaktorköpfe des ersten und/oder zweiten Reaktors weisen bevorzugt die Form einer durchmessererweiterte "Blase" auf, um ein besseres Abströmen der Depolymerisationsprodukte aus der Schmelze und eine Sammlung und Strömungsberuhigung der gasförmigen Depolymerisationprodukte vor dem Ableiten in den Vorkondensator zu ermöglichen. Weiterhin soll die Möglichkeit gegeben sein, dass eventuell beim Abgasen der Depolymerisationsprodukte mitgerissene Schmelzepartikel zurück in die Schmelze gelangen können.

Des Weiteren ist es bevorzugt, wenn der mindestens eine erste Reaktor und der mindestens eine zweite Reaktor von mindestens einem Schutzgas, insbesondere Stickstoff, überlagert werden, so dass der Vorgang des Polymerabbaus unter einer Schutzgasatmosphäre stattfinden kann.

Die vorliegende Anlage und somit auch das vorliegende Verfahren kann bei einem Druck zwischen 0.4 bis 5 bar, bevorzugt zwischen 1 bis 4 bar betrieben werden. Besonders bevorzugt ist ein Betrieb der Anlage unter Normaldruck von 1 bar, so dass eine Druckbeaufschlagung und der damit verbundene apparative Aufwand vermieden werden können.

Des Weiteren ist die vorliegende Anlage inklusive der Rohrleitungen bevorzugt temperaturgeführt und wärmeisoliert.

In einer Variante der vorliegenden Anlage ist zwischen dem mindestens einen zweiten Reaktor und dem mindestens einem Hauptkondensator mindestens ein Vorkondensator z.B. in Form eines Spiralrohrwärmeüberträgers zum Vorkühlen der den mindestens einen zweiten Reaktor verlassenden gasförmigen Kohlenwasserstoffe angeordnet.

Auch ist es bevorzugt, dass der mindestens eine Vorkondensator mit mindestens einem Mischbehälter zur Aufnahme der in dem mindestens einen Vorkondensator während des Vorkühlens der den mindestens einen zweiten Reaktor verlassenden gasförmigen Kohlenwasserstoffe gebildeten Parafine verbunden ist.

Auch verfügt die vorliegende Anlage bevorzugt über mindestens eine Steuereinheit zur Steuerung der Zuführrate der Polyolefine zu dem mindestens einen Extruder. Diese mindestens eine Steuereinheit umfasst bevorzugt jeweils eine Wägeeinrichtung zur Kontrolle des Füllstandes des mindestens einen ersten Reaktors und des mindestens einen zweiten Reaktors. Die Dosierung der Zuführrate der abzubauenden synthetischen Polymere in den Extruder erfolgt bevorzugt über die in dem mindestens einen ersten Reaktor und in dem mindestens einen zweiten Reaktor gemessene Abbaurate.

Die Steuereinheit ermöglicht auch die Steuerung der Temperatur der Anlage. Hierbei werden der Druck vor dem Schmelzefilter, die Temperaturen entlang der heißen Strecken, d.h. entlang der geheizten Rohre zwischen den einzelnen Reaktoren, die einzelnen Temperaturzonen in dem mindestens einen ersten Reaktor und in dem mindestens einen zweiten Reaktor sowie im Mischbehälter gemessen und überwacht. Aus den gemessenen Werten definiert sich die Steuerung und Regelung aller Heizsysteme. Die Steuereinheit erlaubt somit das Fahren bzw. Betreiben der Anlage mit frei wählbaren Temperaturprofilen, die in Abhängigkeit des verwendeten Polymergemisches einstellbar sind.

Es ist ebenfalls möglich, wenn zwischen dem mindestens einen ersten Reaktor und dem mindestens einen zweiten Reaktor mindestens ein Pufferbehälter angeordnet ist, der als Sicherheitsbehälter dient. Der mindestens eine Pufferbehälter wird bevorzugterweise gekühlt, um im Notfall ein rasches Abkühlen der in dem mindestens einen ersten Reaktor und in dem mindestens einen zweiten Reaktor befindlichen Polymerschmelze durch Abführen der selbigen in den Pufferbehälter garantiert. Hierzu ist zwischen der Anlage und dem Pufferbehälter mindestens ein Druckventil eingebaut, das sich bei Verpuffungen öffnet und den Weg für Gase oder auch ggf. Polymerschmelze etc. in dem Behälter freigibt.

In einer weiteren bevorzugten Ausführungsform der vorliegenden Anlage und des vorliegenden Verfahrens werden Kompensatoren z.B. in Form von flexiblen Komponenten z.B. aus Edelstahl verwendet. Die Kompensatoren können beheizbar sein. So können die Kompensatoren z.B. Heizmäntel in Form von abnehmbaren Manschetten und auch zusätzliche Dichtungen in Form von abnehmbaren Schutzmänteln aufweisen.

Wie bereits oben beschrieben, ermöglicht das vorliegende Verfahren, insbesondere bei Durchführung einer fraktionierten Destillation, die Herstellung einer Heizölfraktion und einer Benzinfraktion.

Das hergestellte Heizöl ist durch einen besonders niedrigen Schwefelanteil und einen gegenüber normalem Heizöl erhöhten Flammpunkt gekennzeichnet. So liegt der Flammpunkt eines aus einer PE/PP-Mischung (50:50) gewonnenen Heizöls (nach EN ISO 2719) zwischen 70°C und 90 °C, bevorzugt zwischen 75°C und 90°C, und der Schwefelanteil (nach EN ISO 20846, EN ISO 20884) liegt zwischen 1 und 20 mg pro Kilogramm, bevorzugt zwischen 1 und 15 mg/kg.

Im Vergleich zu dem für das mittels des vom Verfahren hergestellte Heizöl weist ein normales, aus Erdöl gewonnenes Heizöl einen Flammpunkt von 55 bis 60 °C auf.

Die durch das vorliegende Verfahren gewonnene Heizölfraktion weist einen hohen Anteil an C13-C18, insbesondere ungesättigten, Kohlenwasserstoffen auf, wohingegen normales, aus Erdöl gewonnenes Heizöl einen erhöhten Anteil an C11-C13 Kohlenwasserstoffen enthält.

So kann der Anteil an C13-C18 Kohlenwasserstoffen in der mit dem vorliegenden Verfahren gewonnenen Heizölfraktion zwischen 5 und 20 Anteilen /Gew%, bevorzugt 8 und 18 Anteilen / Gew% betragen. Der Anteil an C13-C16 Kohlenwasserstoffen kann sogar zwischen 10 und 20 Anteilen / Gew%, bevorzugt zwischen 11 und 18 Anteilen / Gew% liegen.

Die gewonnene Heizölfraktion kann insbesondere für stationäre Verbrennungsmotoren oder in Kraftwerken verwendet werden.

Die mittels des vorliegenden Verfahrens hergestellte Benzinfraktion weist einen hohen Anteil an niederen, vorwiegend ungesättigten C6-C12 Kohlenwasserstoffen auf und nur geringe Mengen an höheren Kohlenwasserstoffen sind enthalten. Der Anteil an C9-Kohlenwasserstoffen in der Benzinfraktion kann z.B. bei 30-40, bevorzugt 34-38 Anteilen / Gew% liegen. Der Olefingehalt liegt bevorzugt bei Werten größer 90%.

Das bevorzugte Einsatzgebiet der mittels des vorliegenden Verfahrens gewonnenen Benzinfraktion liegt im Bereich von Massenchemikalien bzw. sogenannten Bulk-Chemikalien. So kann die Benzinfraktion aufgrund des hohen Anteils an ungesättigten niederen Kohlenwasserstoffen in der Polymerchemie eingesetzt werden.

Die Erfindung wird im Folgenden ausführlicher anhand eines Ausführungsbeispiels unter Verwendung der Zeichnungen der Figuren beschrieben. Es zeigen:

Figur 1    ein Prozessablaufschema von mindestens einer Ausführungsform des vorliegenden Verfahrens;

Figur 2a    eine schematische Darstellung des Aufbaus von einer ersten Ausführungsform der vorliegenden Anlage;

Figur 2b    eine schematische Darstellung des Aufbaus von einer zweiten Ausführungsform der vorliegenden Anlage;

Figur 3    eine Zusammensetzung eines aus Polypropylen (PP) bei verschiedenen Thermolysetemperaturen gewonnenes Produktöles;

Figur 4    eine Zusammensetzung eines aus Polyethylen (PE) bei verschiedenen Thermolysetemperaturen gewonnenes Produktöles,

Figur 5    eine Darstellung der quantitativen Zusammensetzung der mit dem vorliegenden Verfahren hergestellten Heizölfraktion;

Figur 6    eine Darstellung der quantitativen Zusammensetzung der mit dem vorliegenden Verfahren hergestellten Benzinfraktion,

Figur 7a    eine Ausführungsform eines Rohres mit Stutzen S zur Einspeisung eines eine Tonmineral enthaltenden Gemisches in die Schmelze in der Rohrleitung zwischen ersten und zweitem Reaktor, und

Figur 7b    Draufsicht auf Sichtebene A-A des Stutzen S.

Erstes Ausführungsbeispiel

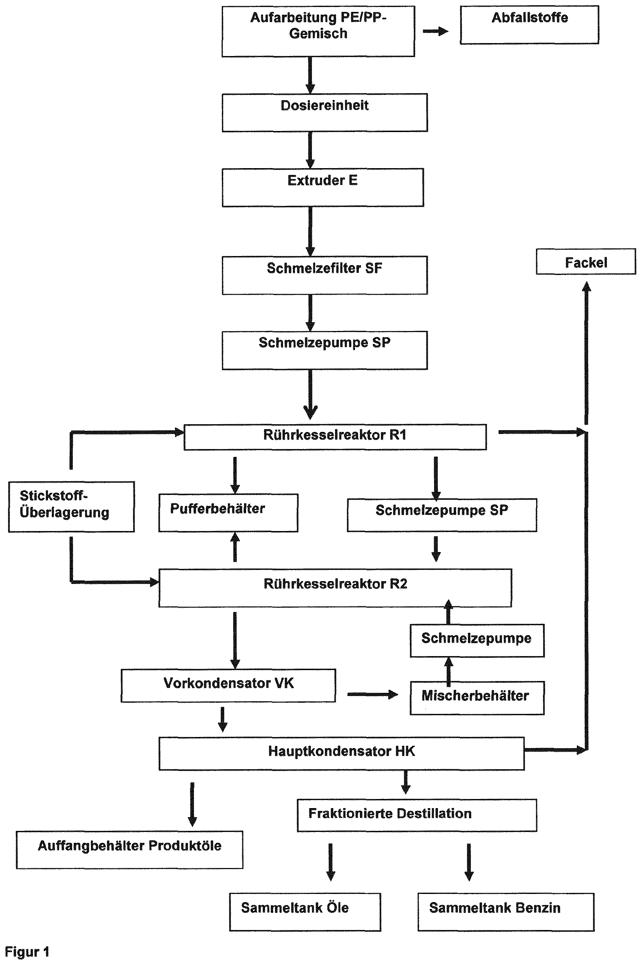

In Figur 1 ist ein Prozessablaufschema einer ersten Ausführungsform des vorliegenden Verfahrens dargestellt. Grundlage des Verfahrens ist ein Polyolefingemisch mit einem Anteil von > 95% an Polyethylen und Polypropylen, das zerkleinert wird und von Abfallstoffen wie Mineralien, Metallen, Faserstoffen, Papieren und Plastik gereinigt wird. Die hierfür bekannten Verfahren sind dem Fachmann bekannt. Die Abfallstoffe werden entweder für den Fall, dass es sich um biogene Abfallstoffe handelt, dem Waschwasser zugeführt oder anderen Entsorgungsmaßnahmen unterzogen.

Das aufgearbeitete PE/PP-Gemisch wird über eine Dosiereinheit als Zuführeinrichtung Z kontinuierlich einem Extruder E zugeführt. In dem Extruder E erfolgt das Aufschmelzen des PE/PP-Gemisches durch Erwärmung auf 300°C, wobei gleichzeitig eine Restfeuchtigkeit aus dem Polymergemisch entfernt wird sowie eine Entgasung erfolgt.

Das im Extruder E aufgeschmolzene PE/PP-Gemisch wird über eine geheizte Rohrleitung in bzw. durch den Schmelzefilter SF transportiert, in welchem die PE/PP-Schmelze von restlichen Spuren an Mineralien, Metallen und Faserstoffen befreit wird.

Die aus dem Schmelzefilter SF austretende hochreine PE/PP-Schmelze wird anschließend kontrolliert mittels einer Schmelzepumpe, die wie alle im Verfahren eingesetzten Schmelzepumpen auch als Rückflußsperre fungiert, in dem unteren Bereich (Sumpfbereich) des ersten Rührkesselreaktor R1 mit einer Temperatur von 300°C eingeführt. Der erste Rührkesselreaktor R1 weist ein maximales Füllvolumen von 120 l auf und ein Verhältnis von Höhe zu Durchmesser von 5,5:1. Der Rührkesselreaktor R1 ist mit fünf separat regelbaren Heizzonen HZ1-HZ5 ausgerüstet und umfasst ein Rührorgan, das eine Kombination aus Wendelrührer und Schnecke darstellt. Der Rührer ist mit einem (mehreren) Temperaturmessfühler(n) im Rührschacht bestückt. Der Sumpfauslass des ersten Reaktors R1 ist beheizt und die Messung des Füllstandes des ersten Reaktors R1 erfolgt über eine Wägeeinrichtung.

Im ersten Rührkesselreaktor R1 wird das PE/P-Polymergemisch schonend und gleichmäßig vom unteren (Sumpfbereich) in den oberen (Kopfbereich) Bereich des Reaktors geführt und dabei stufenweise auf 350°C erhitzt. Die Eingangsviskosität des PE/PP-Polymergemisches im unteren Bereich (Sumpfbereich) des Reaktors R1 liegt bei $1,5*10^6$ MPas (300°C). Aufgrund der stufenweisen Erhitzung des Polymergemisches im Reaktor R1 während des Auftriebes entlang der Wandung des Reaktors wird das Polymergemisch schonend zu Oligomeren abgebaut.

Das im Reaktorkopf des Rührkesselreaktor R1 ankommende Oligomergemisch weist eine Temperatur von 350°C auf und eine Ausgangsviskosität von 671 MPas (350 °C). Neben dem Abbau der PE/PP-Polymere zu den entsprechenden Oligomeren kann es im Rührkesselreaktor R1 ebenfalls zur Bildung von geringen Mengen an gasförmigen Kohlenwasserstoffen kommen, die vorliegend zur Fackel abgeführt werden.

Aus dem Rührkesselreaktor R1 wird das Edukt (Oligomermischung) über eine beheizte Rohrleitung unter Verwendung einer Schmelzepumpe bei 350°C in den unteren Bereich (Sumpfbereich) des Rührkesselreaktors R2 befördert.

Im Rührkesselreaktor R2 wird das Edukt schonend und gleichmäßig von dem unterem Bereich (Sumpfbereich) des Reaktors R2 in den oberen Bereich (Kopfbereich) des Reaktors R2 geführt und bis maximal 410°C erhitzt. Die dabei abnehmende Viskosität generiert einen Auftrieb der Oligomermischung an der Wandung und einen gleichzeitigen Abtrieb im Kern.

Die weitere Aufspaltung des Oligomergemisches aus PE und PP zu kurzkettigen Kohlenwasserstoffen erfolgt zielgenau im oberen Bereich (Kopfbereich) des Reaktors R2, d.h. im Reaktorkopf. Die PE/PP-Oligomermischung, die aus dem Rührkesselreaktor R1 in den Rührkesselreaktor R2 eingeführt wird, weist eine Eingangsviskosität von 671 MPas (350 °C) auf. Aufgrund des weiteren Abbaus bzw. der weiteren Polymerdegradation verringert sich die Viskosität innerhalb des Rührkesselreaktors R2 auf einer Viskosität am Reaktorkopf von 200 MPas (410°C). Der Umsatz des Oligomergemisches zu gasförmigen Kohlenwasserstoffen liegt im zweiten Rührkesselreaktor R2 bei ca. 50l/h.

Die im oberen Bereich des Rührkesselreaktors R2 entstandenen gasförmigen Kohlenwasserstoffe werden unmittelbar in den Vorkondensator VK bzw. Vorabscheider eingeführt und dort auf 350°C abgekühlt. Das schnelle Herunterkühlen der Temperatur der gasförmigen Kohlenwasserstoffe von 410°C auf 350°C unterdrückt weitere ungewollte Folgereaktionen, insbesondere Degradationsreaktionen, durch Abbruch der thermischen Radikalbildung.

Die infolge dessen im Vorkondensator VK abgeschiedenen Parafine, d.h. vorwiegend Alkane mit der allgemeinen Summenformel $C_nH_{2n+2}$, wobei n zwischen 18 und 32, bevorzugt >22 liegt, werden aus dem Vorkondensator VK in den Mischbehälter M abgeführt, in welchem eine Vermengung der Parafine mit Edukt (Oligomermischung) aus dem unteren Bereich (Sumpfbereich) des Rührkesselreaktors R2 erfolgt. Die Temperatur des Mischbehälters M wird mittels Beheizung bei 350°C gehalten. Die Mischung aus Oligomeredukt und Parafinen wird dosiert mit Hilfe einer Schmelzpumpe über eine beheizte Rohrleitung direkt in die Reaktionszone (Reaktorkopf) des Rührkesselreaktors R2. Dieser Prozessschritt verhindert einen weiteren Abbau der Olefinmoleküle, das Aufkommen von Parafinen im Produktöl und insbesondere die Rückführung von degradierbaren Oligomeren/Parafinen in den Abbauprozess und erhöht die Ausbeute an Produktöl.

Die gasförmigen Kohlenwasserstoffe gelangen aus dem Vorkondensator VK in den Hauptkondensator HK und kondensieren dort zu einem Produktöl mit einer Austrittstemperatur von ca. 20 °C. Nicht kondensierbare Gase werden durch eine Gasuhr zur Messung der Volumenströme in die Fackel geleitet. Ebenfalls ist es möglich, diese Gase nicht zu verbrennen, sondern mittels einer Gasturbine zur energetischen Nutzung für die Anlage zu verwenden.

Der Rührkesselreaktor R1 und der Rührkesselreaktor R2 sind jeweils mit Stickstoff überlagert, so dass die Depolymerisation des PE/PP-Ausgangsgemisches vollständig in einer Stickstoffatmosphäre stattfindet und es nicht zu ungewünschten Oxidationen kommt.

Zwischen dem Rührkesselreaktor R1 und dem Rührkesselreaktor R2 ist des Weiteren ein Pufferbehälter angeordnet, der als gekühlter Sicherheitsbehälter dient. Ein zwischen der Anlage und dem Behälter eingebautes Druckventil öffnet sich bei Verpuffungen und gibt den Weg für Gase etc. in den Pufferbehälter frei.

Das aus dem Hauptkondensator gewonnene synthetische Produktöl wird in einer anschließenden fraktionierten Destillation in eine Benzin-Fraktion und eine Heizöl-Fraktion aufgetrennt.

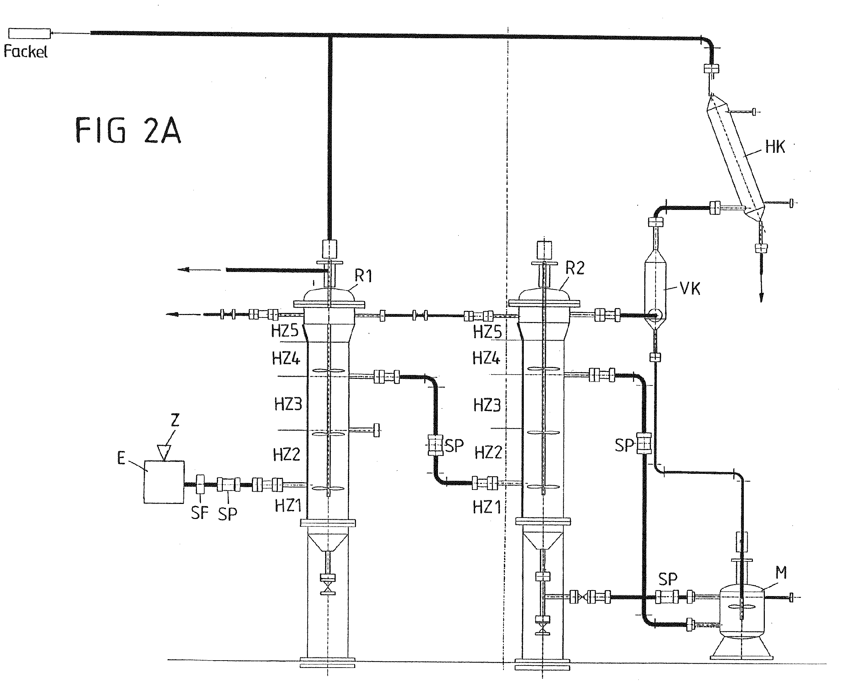

In Figur 2a ist eine schematische Darstellung des Aufbaus einer Anlage gemäß der ersten Ausführungsform dargestellt. Zur Funktion der Anlage wird im Wesentlichen auf die oben für das Prozessschema der Figur 1 angeführte Beschreibung verwiesen.

In der ersten Ausführungsform umfassen sowohl der erste Rührkesselreaktor R1 als auch der zweite Rührkesselreaktor R2 fünf Heizzonen, wobei diese Anzahl auch variieren kann.

Zweites Ausführungsbeispiel

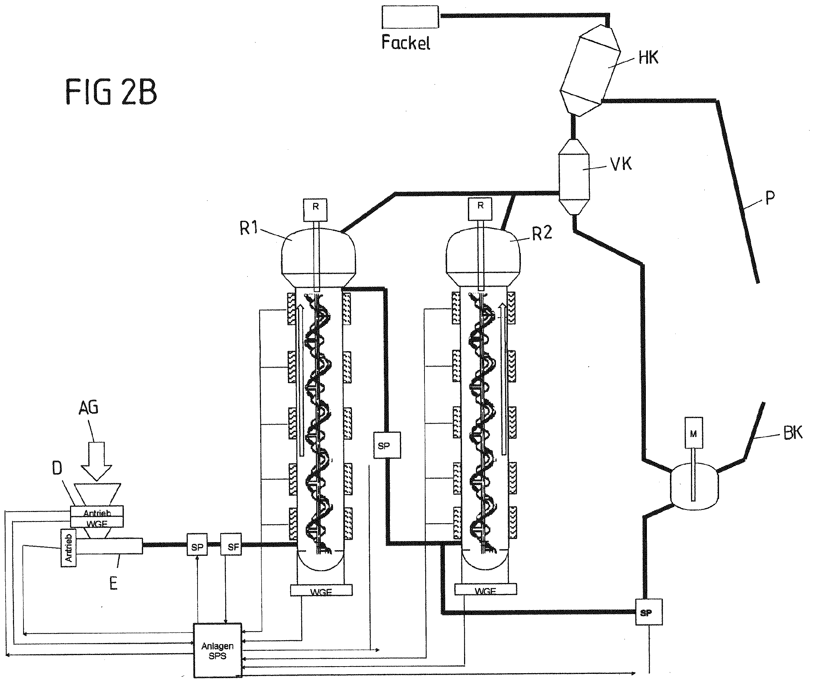

Figur 2b zeigt eine schematische Darstellung einer zweiten Ausführungsform der erfindungsgemäßen Anlage.

Wie oben ausgeführt werden die aufbereiteten Altkunststoffschnitzel einer Voragglomerisierung unterzogen und erlangen damit eine Konsistenz, welche den Eintrag in die Anlage ermöglicht. Dieser voragglomerisierte Altkunststoff AG wird in ein Dosiergerät D mit Schneckenantrieb und Wägeeinrichtung WGE eingegeben. Der Schneckenantrieb und die Wägeeinrichtung WGE sind sowohl als Signalgeber, als auch und Signalempfänger an die Zentrale Anlagen-SPS angeschlossen. In dieser Dosiereinrichtung erfolgt gleichzeitig eine weitere Trocknung und Vorwärmung. Unter der Dosiereinrichtung ist ein geheizter Doppelschneckenextruder E angeordnet, welcher den Rohstoff von 20 °C auf > 200 °C erwärmt, verdichtet und schmilzt. Durch eine geheizte Rohrleitung wird der pastöse Rohstoff weiter aufgewärmt und in eine Schmelzepumpe SP gefördert. Diese Schmelzepumpe SP drückt den Rohstoff in die Anlage und verhindert gleichzeitig den Rücklauf der Schmelze zum Extruder E. Unmittelbar hinter der Schmelzepumpe SP ist der Schmelzefilter SF mit einem feinmaschigen, austauschbaren Gewebefilter angeordnet. Die Funktionsfähigkeit des Schmelzefilters wird kontrolliert durch einen vorgeschalteten Drucksensor, der wiederum mit der Anlagen-SPS verbunden ist.

Die so gereinigte Kunststoffschmelze wird im Sumpfbereich in den ersten Reaktor R1 eingetragen. Die Kunststoffschmelze hat am Eintritt in den Reaktor R1 eine Temperatur von 300 °C und eine Viskosität von 150 Pas. Der Reaktor R1 steht auf einer Wägeeinrichtung WGE, welche zu jeder Zeit den aktuellen Füllstand des Reaktors R1 an die Anlagen-SPS meldet. Der Reaktor R1 ist mit einer keramischen Schicht ausgekleidet, um den direkten Kontakt der Schmelze zur metallischen Reaktorwand zu verhindern. Der Reaktor R1 ist als schlanker Reaktor mit einem Durchmesser/Längen-Verhältnis von 1:5,5 bis 1:7 ausgeführt.

An der zylindrischen Wandung des Reaktors R1 sind 3 bis 5 unabhängig voneinander wirkende Heizungen angebracht. In jeder zugehörigen Heizzone sind Temperatursensoren angeordnet, welche die jeweils aktuell wirkende Temperatur an der Wandung des Reaktors R1 in dieser Heizzone an die Anlagen-SPS melden. Die Anlagen-SPS steuert die Heizungen so, dass im zylindrischen Teil des Reaktors R1 von Sumpf zum Kopf ein kontinuierlich steigender Temperaturgradient entsteht. Durch diese kontinuierlich steigende Temperatur im Reaktor R1 wird eine kontinuierlich nach oben strömende laminare Schmelzeströmung erzwungen.

Die nach oben gerichtete laminare Schmelzeströmung wird durch einen Rührer unterstützt, dessen Flügel so gestaltet sind, dass die Strömung nach oben unterstützt wird. Mit dieser Strömungsgestaltung wird eine mögliche Depolymerisation in den Kopfbereich des Reaktors R1 verlegt. Eventuell bereits entstehende gasförmige Depolymerisationsprodukte werden sofort durch die laminare Strömung in den Kopfbereich gefördert und somit ein Aufschäumen der Schmelze verhindert.

Am Kopf des Reaktors R1 erreicht die Schmelze eine Temperatur von 340 °C bis 360 °C und eine Viskosität von 1,5 Pas. Durch dieses Temperaturregime und durch die Zuspeisung eines Bentonitkatalysaors erst in den Reaktor R2 wird im Reaktor 1 nur ein geringer Teil der Schmelze sich in gasförmige Depolymerisationsprodukte umsetzen. Die restliche Schmelze erfährt eine Vorspaltung. Die im Reaktor R1 entstehenden gasförmigen Depolymerisationsprodukte werden über eine geheizte Rohrleitung in den Vorkondensator geleitet.

Vom Kopfbereich des Reaktors R1 wird die Schmelze, über eine geheizte Rohrleitung und eine Schmelzepumpe SP in den Reaktor R2 geleitet. Die Schmelze tritt mit einer Temperatur von 350 °C und einer Viskosität von 1,5 Pas in der Sumpfzone des Reaktors R2 in diesen ein.

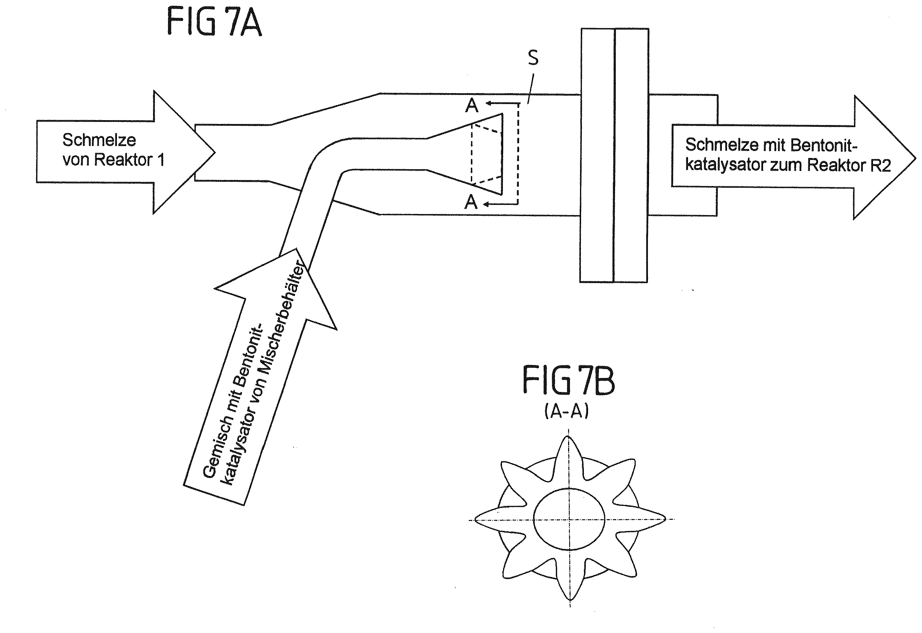

Beim Eintritt der Schmelze in den Reaktor R2 wird diese durch einen speziell gestalteten Stutzen mit dem vom Mischbehälter M kommenden Gemisch aus Bentonitkatalysator, Paraffinen und Mikrowachsen vermischt. Dieser speziell gestaltete Stutzen ist als "Hosenrohr" ausgeführt, wobei die Zuführung des Gemisches, welches aus dem Mischbehälter M kommt in die Kernzone des Rohres, in welchem die Schmelze aus dem Reaktor R1 zugeführt wird, geführt ist (siehe hierzu auch Figur 7a, b).

Der Reaktor R2 steht auf einer Wägeeinrichtung WGE, welche zu jeder Zeit den aktuellen Füllstand des Reaktors 2 an die Anlagen-SPS meldet. Der Reaktor R2 ist ebenfalls mit einer keramischen Schicht ausgekleidet, um den direkten Kontakt der Schmelze zur metallischen Reaktorwand zu verhindern. Der Reaktor 2 ist als schlanker Reaktor mit einem Durchmesser/Längenverhältnis von 1:5,5 bis 1:7 ausgeführt.

An der zylindrischen Wandung des Reaktors 2 sind 3 bis 5 unabhängig voneinander wirkende Heizungen angebracht. In jeder Heizzone sind Temperatursensoren angeordnet, welche die jeweils aktuell wirkende Temperatur an der Wandung des Reaktors 2 in dieser Heizzone an die Anlagen-SPS melden. Die Anlagen-SPS steuert die Heizungen so, dass im zylindrischen Teil des Reaktors R2 von Sumpf zum Kopf ein kontinuierlich steigender Temperaturgradient entsteht. Durch diese kontinuierlich steigende Temperatur im Außenbereich des Reaktors R2 wird eine kontinuierlich nach oben strömende laminare Schmelzeströmung erzwungen. Die nach oben gerichtete laminare Schmelzeströmung wird durch einen Rührer unterstützt, dessen Flügel so gestaltet sind, dass die Strömung im Außenbereich des Reaktors R2 nach oben und im Innenbereich des Reaktors R2 eine nach unten gerichtete Strömung, unterstützt wird. Mit dieser Strömungsgestaltung wird die Depolymerisation in den Kopfbereich des Reaktors R2 verlegt. Entstehende gasförmige Depolymerisationsprodukte werden sofort durch die laminare Strömung in den Kopfbereich gefördert und somit ein Aufschäumen der Schmelze verhindert.

Am Kopf des Reaktors R2 erreicht die Schmelze eine Temperatur von 400 °C bis 410 °C und eine Viskosität von 0,1 Pas. Eventuell noch nicht depolymerisierte Schmelzeanteile werden durch die durch die Wirkung des speziell gestalteten Rührers im Zentrum des Reaktors 2 nach unten in den Sumpfbereich des Reaktors 2 transportiert und nehmen dann in der äußeren laminaren Aufwärtsströmung erneut an der Depolymerisation teil.

Die Informationen aus den Wägeeinrichtungen WGE der Reaktoren R1, R2 werden in der Anlagen-SPS in Befehle an die Antriebe der Dosiereinrichtung D, des Doppelschneckenextruders E und der Schmelzepumpen SP so umgesetzt, dass die vorliegende Anlage kontinuierlich arbeiten kann.

Die Kopfzonen der Reaktoren R1, R2 werden als durchmessererweiterte "Blase" gestaltet, um ein besseres Abströmen der Depolymerisationsprodukte aus der Schmelze und eine Sammlung und Strömungsberuhigung der gasförmigen Depolymerisationprodukte vor dem Ableiten in den Vorkondensator zu ermöglichen. Weiterhin soll die Möglichkeit gegeben sein, dass eventuell beim Abgasen der Depolymerisationsprodukte mitgerissene Schmelzepartikel zurück in die Schmelze gelangen können.

Die im Reaktor R2 entstehenden gasförmigen Depolymerisationsprodukte der Kettenlängen C3 bis > C22 werden über eine geheizte Rohrleitung in den Vorkondensator VK geleitet. Der Vorkondensator VK ist als Spiralrohrwärmeübertrager ausgeführt. In diesem Vorkondensator VK werden die flüchtigen Depolymerisationsprodukte der Kettenlängen oberhalb C22 auskondensiert. Diese Paraffine, Mikrowachse und Kohlenwasserstoffketten oberhalb C22 werden als pastöse oder flüssige Phase, als systemrelevante Zwischenprodukte, über eine geheizte Rohrleitung, in einen Mischbehälter M nach unten abgeleitet.

Die am Kopf des Vorkondensators VK noch flüchtigen Depolymerisationsbestandteile der Kettenlängen C3 bis C22 werden in den darüber liegenden Hauptkondensator HK geführt. Dieser Hauptkondensator HK ist als schrägliegender Spiralrohrwärmeübertrager ausgeführt. Die Schräglage von 20 °C des Hauptkondensators ist für die optimale Trennung der gasförmigen und flüssigen Phase und deren Ableitung nach unten gewählt. Die Temperaturen im Kopf des Hauptkondensators HK werden mit 20 °C bis 25 °C eingestellt. Damit werden Restgase und flüchtige Bestandteile der Depolymerisationsprodukte der Kettenlängen C3 bis C6 als gasförmige Bestandteile oben aus dem Hauptkondensator austreten. Dieses Abgas wird über eine geheizte Rohrleitung zur Fackelanlage geführt und dort verbrannt. Das aus dem Hauptkondensator HK nach unten in einen Produktsammelbehälter P abgeleitete Produkt ist ein synthetisches Produktöl und besteht aus gesättigten und ungesättigten Kohlenwasserstoffen in einem Siedebereich von 40 °C bis 350 °C und umfasst die Kohlenwasserstofffraktionen von C7 bis C22.

Der Mischbehälter M ist als Rührmaschine ausgeführt, welche bei einer konstanten Temperatur, durch Heizung/Kühlung, von 340 °C bis 350°C arbeitet, bei welcher das Kondensat aus dem Vorkondensator VK in pastösem Zustand gehalten wird. In dieses pastöse Kondensat wird unter ständigem Rühren der pulverförmige Bentonitkatalysator BK eingemischt. Dieses pastöse Gemisch aus Kondensat und Bentonitkondensator wird über eine geheizte Rohrleitung und eine Schmelzepumpe SP in den Reaktor 2 eingetragen Dieser Eintrag erfolgt über den vorstehend bereits beschriebenen hosenförmigen Spezialstutzen (siehe auch Figuren 7a, b).

Figuren 7a,b zeigen den detaillierten Aufbau des hosenförmigen Spezialstutzens S und deren Anordnung in der Rohrleitung zwischen ersten Reaktor R1 und zweiten Reaktor R2. Der Spezialstutzen S ist fächerförmig ausgebildet. Diese spezifische fächerförmige Ausbildung des Stutzens S erlaubt eine optimale Vermischung der Oligomermischung mit der das Tonmineral enthaltenden Mischung aus dem Mischbehälter M.

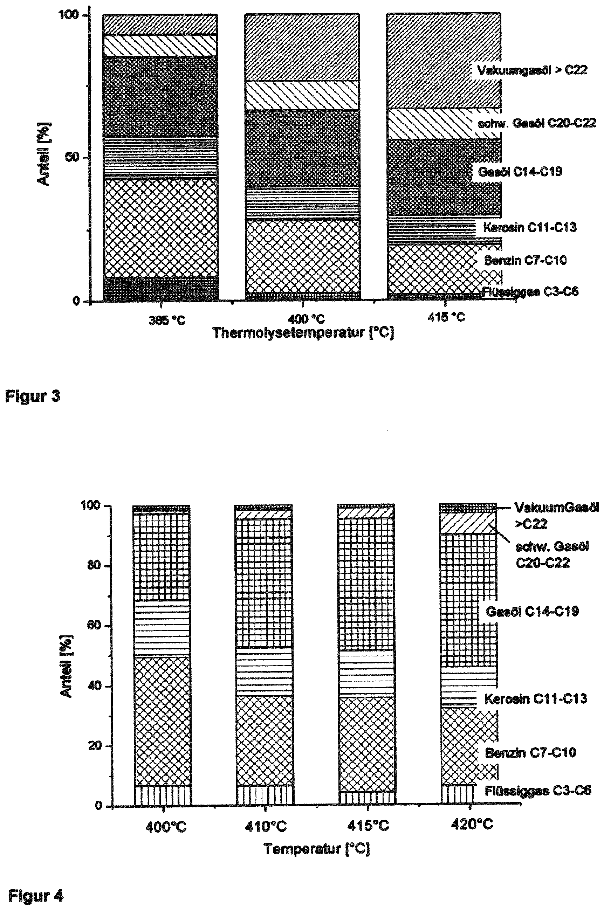

Figur 3 ist ein Diagramm, aus welchem die Zusammensetzung von aus PP gewonnenen Kondensaten bzw. Produktölen in Abhängigkeit von der im zweiten Reaktor R2 angewendeten Thermolysetemperatur dargestellt ist.

Die bei PP-Abbau erhaltenen Komponenten stellen fast ausschließlich oligomere Einheiten von Propen dar und entstehen beim Abbau des Polypropylens durch Spaltung von C-C Bindungen. Die Produkte erstrecken sich über einen weiten Kettenlängenbereich von C3-C30. Als Hauptspaltprodukt wurde bei allen Versuchen unabhängig von der Thermolysetemperatur 2,4-Dimethyl-Hept-1-en gefunden.

Insgesamt können die Komponenten in 6 Fraktionen unterteilt werden: Flüssiggas (C3-C6), Benzin (C7-C10), Kerosin (C11-C13), Gasöl (C14-C19), schweres Gasöl (C20-C22) und Paraffine/Mikrowachse (>C22).

Wie aus dem Diagramm der Figur 3 ersichtlich ist, fallen bei höheren Thermolysetemperaturen mehr Schwersieder (höhere Kohlenwasserstoffe) im Kondensat an. Gleichzeitig sinkt der Anteil an Leichtsiedern (niederen Kohlenwasserstoffen). Besonders deutlich wird das an den Benzin- und Vakuumgasöl (Paraffine/Mikrowachse)-Fraktionen.

Während die Benzinfraktion bei 385°C mit ca. 30% dominiert und der Anteil an Vakuumgasöl verschwindend gering ist, dominiert bei 410°C die Paraffine/Mikrowachse-Fraktion mit einem ähnlichen Anteil und die Benzinfraktion ist nur noch halb so groß. Die mittleren Fraktionen Kerosin, Gasöl und schweres Gasöl bleiben annähernd konstant. Über die Variation der Thermolysetemperatur ist es also in gewissem Maße möglich, die Produktverteilung hin zu einer gewünschten Produktgruppe zu lenken.

So weist ein bei einer Thermolysetemperatur von 385°C gewonnenes PP-Produktöl z.B. folgende Zusammensetzung auf: ca. 10 Gew% Paraffine/Mikrowachse > C22; ca. 10 Gew% schweres Gasöl C20-C22; ca. 25 Gew% Gasöl C14-C19; ca. 15 Gew% Kerosin C11-C13; ca. 30 Gew% Benzin C7-C10 und ca. 10 Gew% Flüssiggas C3-C6.

Ein bei einer Thermolysetemperatur von 400°C gewonnenes PP-Produktöl weist z.B. folgende Zusammensetzung auf: ca. 25 Gew% Paraffine/Mikrowachse > C22; ca. 10 Gew% schweres Gasöl C20-C22; ca. 20 Gew% Gasöl C14-C19; ca. 15 Gew% Kerosin C11-C13; ca. 25 Gew% Benzin C7-C10 und ca. 5 Gew% Flüssiggas C3-C6.

Ein bei einer Thermolysetemperatur von 415°C gewonnenes PP-Produktöl weist z.B. folgende Zusammensetzung auf: ca. 30 Gew% Paraffine/Mikrowachse > C22; ca. 10 Gew% schweres Gasöl C20-C22; ca. 25 Gew% Gasöl C14-C19; ca. 10 Gew% Kerosin C11-C13; ca. 20 Gew% Benzin C7-C10 und weniger als ca. 5 Gew% Flüssiggas C3-C6.

Das in Figur 4 gezeigte Diagramm bezieht sich auf die Zusammensetzung von Kondensaten bzw. Produktölen gewonnenen aus PE. Die aus PE gewonnenen Produkte erstrecken sich etwa über einen Kettenlängenbereich von C3-C30. Die quantitative Analyse der erhaltenen Kondensate ergab, dass die Verteilung der einzelnen Heizwertfraktionen auch beim Einsatz von PE in Abhängigkeit von der Thermolysetemperatur, bei der die Kondensate erhalten wurden, variiert. Wie aus Figur 4 ersichtlich nimmt der Anteil an Gasöl (C14-C19) mit steigender Temperatur zu, während der Anteil der niederen Kohlenwasserstofffraktionen wie Kersosin (C11-C13) und Benzin (C7-C10) abnimmt.

Ein aus Polyethylen (PE) gewonnenes Kondensat umfasst bevorzugt Kohlenwasserstoffe in einem Kettenlängenbereich von C3-C30. Das PE-Kondensat umfasst typischerweise n-Alkane und n-Alkene (Olefine) in einem Verhältnis von 50:50. So liegen die Kohlenwasserstoffe parallel immer in der gesättigten und ungesättigten Form vor wie z.B. C10: n-Undecen und Undec-1-en.

Ein bei einer Thermolysetemperatur von 400°C gewonnenes PE-Produktöl weist z.B. folgende Zusammensetzung auf: Spuren von Paraffine/Mikrowachse > C22; Spuren von schwerem Gasöl C20-C22; ca. 30 Gew% Gasöl C14-C19; ca. 20 Gew% Kerosin C11-C13; ca. 45 Gew% Benzin C7-C10 und ca. 5 -10 Gew% Flüssiggas C3-C6.

Ein bei einer Thermolysetemperatur von 410°C gewonnenes PE-Produktöl weist z.B. folgende Zusammensetzung auf: Spuren von Paraffine/Mikrowachse > C22; weniger als 5 Gew% schweres Gasöl C20-C22; ca. 45 Gew% Gasöl C14-C19; ca. 15 Gew% Kerosin C11-C13; ca. 30 Gew% Benzin C7-C10 und ca. 5 -10 Gew% Flüssiggas C3-C6.

Ein bei einer Thermolysetemperatur von 415°C gewonnenes PE-Produktöl weist z.B. folgende Zusammensetzung auf: Spuren von Paraffine/Mikrowachse > C22; weniger als 5 Gew% schweres Gasöl C20-C22; ca. 45 Gew% Gasöl C14-C19; ca. 15 Gew% Kerosin C11-C13; ca. 30 Gew% Benzin C7-C10 und ca. 5 -10 Gew% Flüssiggas C3-C6.

Ein bei einer Thermolysetemperatur von 420°C gewonnenes PE-Produktöl weist z.B. folgende Zusammensetzung auf: ca. 5 Gew% Paraffine/Mikrowachse > C22; ca. 10 Gew% schweres Gasöl C20-C22; ca. 45 Gew% Gasöl C14-C19; ca. 15 Gew% Kerosin C11-C13; ca. 25 Gew% Benzin C7-C10 und ca. 5 -10 Gew% Flüssiggas C3-C6.

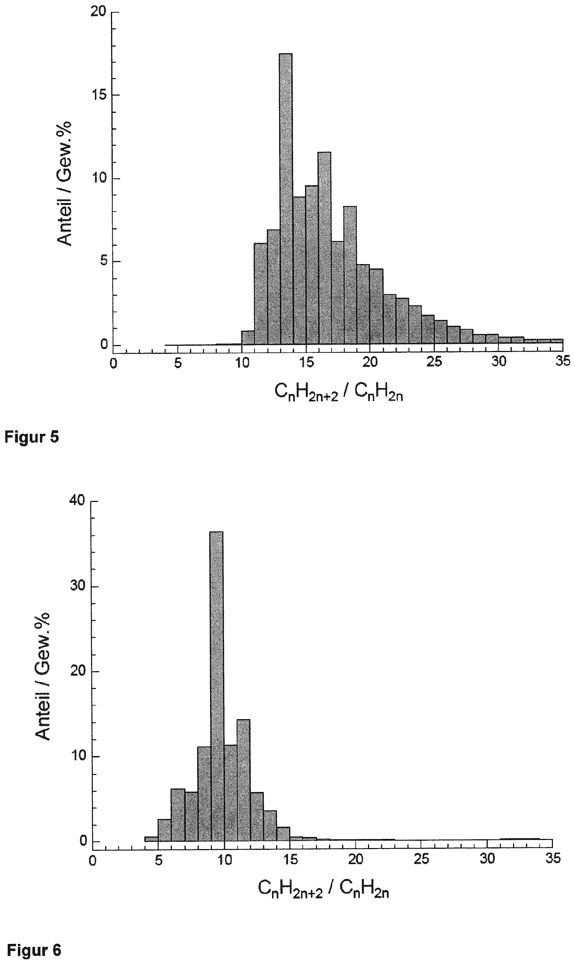

Aus den Figuren 5 und 6 sind jeweils die quantitativen Zusammensetzungen der nach fraktionierter Destillation gewonnen Heizöl-Fraktion und Benzin-Fraktion entnehmbar.

So weist die durch das vorliegende Verfahren gewonnene Heizölfraktion einen hohen Anteil an C13-C18, insbesondere ungesättigten, Kohlenwasserstoffen auf (Figur 5). In dem vorliegenden Beispiel ist der Anteil an C13-Kohlenwasserstoffen mit ca. 18 Gew % besonders hoch. Auch C14-C18 Kohlenwasserstoffe sind mit Anteilen zwischen 8 und 12 Gew% vertreten, während die Kohlenwasserstoffe mit C< 11 nur in sehr geringen Mengen oder überhaupt nicht nachweisbar sind.

Aus Figur 6 ist die quantitative Zusammensetzung einer beispielhaften Benzinfraktion entnehmbar. So umfasst die Benzinfraktion in nennenswerten Mengen C6-C12 Kohlenwasserstoffe, wobei der Anteil an C8-C11 Kohlenwasserstoffen erhöht ist. So liegt der Anteil an C9-Kohlenwasserstoffen z.B. bei ca. 36 Gew %.

**Patentansprüche**

1. Verfahren zum Abbau von synthetischen Polyolefinen,

umfassend die Schritte

a) Herstellen einer Schmelze aus synthetischen Polyolefinen, insbesondere aus trockenen synthetischen Polyolefinen,

b) Reinigen der Polyolefinschmelze mittels Durchleiten der Polyolefinschmelze durch mindestens einen Schmelzefilter (SF),

c) Überführen der gereinigten Polyolefinschmelze in mindestens einen ersten Reaktor (R1), wobei die gereinigte Polyolefinschmelze in dem mindestens einen ersten Reaktor (R1) von einem unteren Bereich (Sumpfbereich) in einen oberen Bereich (Kopfbereich) des Reaktors (R1) unter Erhitzen auf Temperaturen zwischen 300°C und 370°C, bevorzugt 330°C bis 360°C, insbesondere bevorzugt 350°C geführt wird, wobei die Polyolefine in dem mindestens einen ersten Reaktor (R1) in Oligomere gespalten werden,

d) Überführen der in dem mindestens eine ersten Reaktor (R1) gebildeten Oligomermischung in mindestens einen zweiten Reaktor (R2), wobei das Oligomergemisch in dem mindestens zweiten Reaktor (R2) von einem unteren Bereich (Sumpfbereich) in einen oberen Bereich (Kopfbereich) des Reaktors (R2) unter Erhitzen bis auf 380°C bis 450°C, bevorzugt 400°C bis 430°C, insbesondere 410°C geführt wird, wobei die Oligomere in dem mindestens einem zweiten Reaktor (R2) in Gegenwart von mindestens einem Tonmineral als Depolymerisationskatalysator in kurzkettige Kohlenwasserstoffe abgebaut werden,

e) Abführen der in dem mindestens einen zweiten Reaktor (R2) gebildeten kurzkettigen Kohlenwasserstoffen mit C3-C22 in mindestens einen Vorkondensator (VK), wobei die aus dem mindestens einen zweiten Reaktor (R2) austretenden kurzkettigen Kohlenwasserstoffe in dem mindestens einen Vorkondensator (VK) abgekühlt werden; und

f) Einführen der in dem mindestens einem Vorkondensator (VK) abgekühlten kurzkettigen Kohlenwasserstoffe in mindestens einen Hauptkondensator (HK), wobei die aus dem mindestens einem Vorkondensator (VK) austretenden kurzkettigen Kohlenwasserstoffe in dem mindestens einen Hauptkondensator (HK) verflüssigt werden.

2. Verfahren nach Anspruch 1, **dadurch gekennzeichnet, dass** Polyolefin-Gemische aus Polyethylen (PE) und Polypropylen (PP) abgebaut werden.

3. Verfahren nach Anspruch 1 oder 2, **dadurch gekennzeichnet, dass** die Polyolefine in mindestens einen Extruder (E), insbesondere in einem Doppelschneckenextruder, bei Temperaturen von bis zu 300°C, insbesondere bei Temperaturen zwischen 200°C bis 300°C aufgeschmolzen werden.

4. Verfahren nach einem der vorhergehenden Ansprüche, **dadurch gekennzeichnet, dass** in dem mindestens ersten Reaktor (R1) ein Abbau der Polyolefine mit einem typischen Molekulargewicht von größer $10^5$ kg/mol in Oligomere mit einem Molekulargewicht zwischen $10^2$ bis $10^4$ kg/mol, bevorzugt $10^3$ bis $10^4$ kg/mol erfolgt.

5. Verfahren nach einem der vorhergehenden Ansprüche, **dadurch gekennzeichnet, dass** das den mindestens einen ersten Reaktor (R1) verlassene Oligomergemisch vor Einführen in den mindestens einen zweiten Reaktor (R2) mit dem mindestens einem Tonmineral als Depolymerisationskatalysator vermischt wird.

6. Verfahren nach einem der vorhergehenden Ansprüche, **dadurch gekennzeichnet, dass** das mindestens eine Tonmineral ausgewählt ist aus der Gruppe enthaltend Schichtsilikate, insbesondere Montmorillonit, Illit, Bentonit, Kaolinit, Smektit, Chlorit, Vermikulit und Glimmer.

7. Verfahren nach einem der vorhergehenden Ansprüche, **dadurch gekennzeichnet, dass** in dem mindestens einen zweiten Reaktor (R2) die Spaltung der Oligomere in kurzkettige gasförmige Kohlenwasserstoffe mit einem Molekulargewicht kleiner 500 kg/mol erfolgt.

8. Anlage zur Durchführung eines Verfahrens nach einem der vorgehenden Ansprüche umfassend

    - mindestens einen Extruder (E) zum Schmelzen der Polyolefine,
    - mindestens einen stromabwärts von dem mindestens einen Extruder (E) angeordneten Schmelzefilter (SF) zur Reinigung der Polyolefinschmelze,
    - mindestens einen stromabwärts von dem mindestens einen Schmelzefilter (SF) angeordneten ersten Reaktor (R1) zum Abbau der Polyolefine in Oligomere,
    - mindestens einen stromabwärts von dem mindestens einen ersten Reaktor (R1) angeordneten mindestens zweiten Reaktor (R2) zum Abbau der in dem mindestens ersten Reaktor (R1) gebildeten Oligomere in kurzkettige Kohlenwasserstoffe,
    - mindestens einen stromabwärts von dem mindestens einen zweiten Reaktor (R2) angeordneten Vorkondensator (VK) zum Vorkühlen der den zweiten Reaktor (R2) verlassenen kurzkettigen gasförmigen Kohlenwasserstoffe, und

    - mindestens einen stromabwärts von dem mindestens einen Vorkondensator (VK) angeordneten Hauptkondensator (HK) zur Kondensation der in dem mindestens einen Vorkondensator (VK) vorgekühlten kurzkettigen Kohlenwasserstoffe.

9. Anlage nach Anspruch 8, **dadurch gekennzeichnet, dass** der mindestens eine erste Reaktor (R1) und der mindestens eine zweite Reaktor (R2) jeweils über mindestens zwei regelbare Heizzonen, bevorzugt über mindestens fünf regelbare Heizzonen verfügen.

10. Anlage nach Anspruch 8 oder 9, **dadurch gekennzeichnet, dass** der mindestens eine erste Reaktor (R1) und der mindestens eine zweite Reaktor (R2) jeweils über ein Rührorgan in Form eines Wendelrührers, Ankerrührers, Schnecke oder eine Kombination davon, bevorzugt in Form einer Kombination aus Wendelrührer und Schnecke verfügt.

11. Anlage nach einem der Ansprüche 8 bis 10, **dadurch gekennzeichnet, dass** die Innenwände und Einbauten des mindestens einen ersten Reaktors (R1) und/oder des mindestens einen zweiten Reaktors (R2) mindestens eine keramische Beschichtung aufweisen.

12. Anlage nach einem der Ansprüche 8 bis 11, **dadurch gekennzeichnet, dass** der mindestens eine erste Reaktor (R1) und der mindestens eine zweite Reaktor (R2) jeweils ein Verhältnis von Höhe:Durchmesser (H:D) von 7:1, bevorzugt 5,5:1 aufweisen.

13. Anlage nach einem der Ansprüche 8 bis 12, **dadurch gekennzeichnet, dass** der mindestens eine Vorkondensator (VK) mit mindestens einem Mischbehälter (M) zur Aufnahme der in dem mindestens einen Vorkondensator (VK) während des Vorkühlens der den zweiten Reaktor (R2) verlassenden kurzkettigen Kohlenwasserstoffe gebildeten Paraffine verbunden ist.

14. Anlage nach einem der Ansprüche 8 bis 13, **gekennzeichnet durch** mindestens eine Steuereinheit zur Steuerung der Zuführrate der Polyolefine zu dem mindestens einen Extruder (E) und zur Steuerung der Temperatur der Anlage.

15. Produktöl hergestellt in einem Verfahren nach einem der Ansprüche 1 bis 7 umfassend die Fraktionen Flüssiggas (C3-C6), Benzin (C7-C10), Kerosin (C11-C13), Gasöl (C14-C19), schweres Gasöl (C20-C22) und Paraffine/Mikrowachse (>C22).

**Claims**

1. A method for breaking down polyolefins, comprising the steps

   a) producing a melt of synthetic polyolefins, in particular of dry synthetic polyolefins,
   b) purifying the polyolefin melt by passing the polyolefin melt through at least one melt filter (SF),
   c) transferring the purified polyolefin melt into at least one first reactor (R1), wherein the purified polyolefin melt in the at least one first reactor (R1) is conducted from a lower region (sump region) to an upper region (head region) of the reactor (R1) with heating to temperatures between 300°C and 370°C, preferably 330°C to 360°C, particularly preferably 350°C, wherein the polyolefins in the at least one first reactor (R1) are cleaved into oligomers,
   d) transferring the oligomer mixture formed in the at least one first reactor (R1) to at least one second reactor (R2), wherein the oligomer mixture in the at least second reactor (R2) is conducted from a lower region (sump region) to an upper region (head region) of the reactor (R2) with heating to 380°C to 450°C, preferably 400°C to 430°C, in particular 410°C, wherein the oligomers in the at least one second reactor (R2) are broken down to short-chain hydrocarbons in the presence of at least one clay mineral as depolymerization catalyst,
   e) removing the short-chain hydrocarbons having C3-C22 that are formed in the at least one second reactor (R2) to at least one precondenser (VK), wherein the short-chain hydrocarbons exiting from the at least one second reactor (R2) are cooled in the at least one precondenser (VK); and
   f) introducing the short-chain hydrocarbons that are cooled in the at least one precondenser (VK) into at least one main condenser (HK), wherein the short-chain hydrocarbons exiting from the at least one precondenser (VK) are liquefied in the at least one main condenser (HK).

2. The method as claimed in claim 1, **characterized in that** polyolefin mixtures of polyethylene (PE) and polypropylene (PP) are broken down.

3. The method as claimed in claim 1 or 2, **characterized in that** the polyolefins are melted in at least one extruder (E), in particular in a twin-screw extruder, at temperatures of up to 300°C, in particular at temperatures between 200°C and 300°C.

4. The method as claimed in any one of the preceding claims, **characterized in that** the polyolefins having a typical molecular weight of greater than $10^5$ kg/mol are broken down into oligomers having a molecular weight between $10^2$ and $10^4$ kg/mol, preferably $10^3$ to $10^4$ kg/mol, in the at least first reactor (R1).

5. The method as claimed in any one of the preceding claims, **characterized in that** the oligomer mixture leaving the at least one first reactor (R1) is mixed with the at least one clay mineral as depolymerization catalyst before it is introduced into the at least one second reactor (R2).

6. The method as claimed in any one of the preceding claims, **characterized in that** the at least one clay mineral is selected from the group containing layer silicates, in particular montmorillonite, illite, bentonite, kaolinite, smectite, chlorite, vermiculite and mica.

7. The method as claimed in any one of the preceding claims, **characterized in that** the oligomers are cleaved into short-chain gaseous hydrocarbons having a molecular weight less than 500 kg/mol in the at least one second reactor (R2).

8. A system for carrying out a method as claimed in any one of the preceding claims, comprising

   - at least one extruder (E) for melting the polyolefins,
   - at least one melt filter (SF) that is for purifying the polyolefin melt and is arranged downstream of the at least one extruder (E),
   - at least one first reactor (R1) for breaking down the polyolefins into oligomers that is arranged downstream of the at least one melt filter (SF),
   - at least one second reactor (R2) arranged downstream of the at least one first reactor (R1) for breaking down the oligomers formed in the at least first reactor (R1) into short-chain hydrocarbons,
   - at least one precondenser (VK) arranged downstream of the at least one second reactor (R2) for precooling the short-chain gaseous hydrocarbons leaving the second reactor (R2), and
   - at least one main condenser (HK) arranged downstream of the at least one precondenser (VK) for condensation of the short-chain hydrocarbons that are precooled in the at least one precondenser (VK).

9. The system as claimed in claim 8, **characterized in that** the at least one first reactor (R1) and the at least one second reactor (R2) each have at least two controllable heating zones, preferably at least five controllable heating zones.

10. The system as claimed in claim 8 or 9, **characterized**

**in that** the at least one first reactor (R1) and the at least one second reactor (R2) each have an agitator element in the form of a spiral agitator, anchor agitator, screw or a combination thereof, preferably in the form of a combination of spiral agitator and screw.

11. The system as claimed in any one of claims 8 to 10, **characterized in that** the inner walls and internals of the at least one first reactor (R1) and/or of the at least one second reactor (R2) have at least one ceramic coating.

12. The system as claimed in any one of claims 8 to 11, **characterized in that** the at least one first reactor (R1) and the at least one second reactor (R2) each have a height:diameter (H:D) ratio of 7:1, preferably 5.5:1.

13. The system as claimed in any one of claims 8 to 12, **characterized in that** the at least one precondenser (VK) is connected to at least one mixing vessel (M) for receiving the paraffins formed in the at least one precondenser (VK) during the precooling of the short-chain hydrocarbons leaving the second reactor (R2).

14. The system as claimed in any one of claims 8 to 13, **characterized by** at least one control unit for controlling the feed rate of the polyolefins to the at least one extruder (E) and for controlling the temperature of the system.

15. A product oil produced in a method as claimed in any one of claims 1 to 7 comprising the fractions of liquid gas (C3-C6), gasoline (C7-C10), kerosene (C11-C13), gas oil (C14-C19), heavy gas oil (C20-C22) and paraffins/microwaxes (>C22).

## Revendications

1. Procédé de décomposition de polyoléfines synthétiques,
comprenant les étapes suivantes:

a) la fabrication d'une masse fondue de polyoléfines synthétiques, notamment de polyoléfines synthétiques sèches,
b) la purification de la masse fondue de polyoléfines par passage de la masse fondue de polyoléfines dans au moins un filtre à masse fondue (SF),
c) le transfert de la masse fondue de polyoléfines purifiée dans au moins un premier réacteur (R1), la masse fondue de polyoléfines purifiée étant conduite dans l'au moins un premier réacteur (R1) depuis une zone inférieure (zone de fond) dans une zone supérieure (zone de tête) du réacteur (R1) avec chauffage à des températures comprises entre 300 °C et 370 °C, de préférence 330 °C et 360 °C, de manière particulièrement préférée de 350 °C, les polyoléfines étant clivées en oligomères dans l'au moins un premier réacteur (R1),
d) le transfert du mélange d'oligomères formé dans l'au moins un premier réacteur (R1) dans au moins un deuxième réacteur (R2), le mélange d'oligomères étant conduit dans l'au moins un deuxième réacteur (R2) depuis une zone inférieure (zone de fond) dans une zone supérieure (zone de tête) du réacteur (R2) avec chauffage jusqu"à des températures de 380 °C à 450 °C, de préférence de 400 °C à 430 °C, notamment de 410 °C, les oligomères étant décomposés en hydrocarbures à chaîne courte dans l'au moins un deuxième réacteur (R2) en présence d'au moins un minéral argileux en tant que catalyseur de dépolymérisation,
e) le transport des hydrocarbures à chaîne courte en C3-C22 formés dans l'au moins un deuxième réacteur (R2) dans au moins un pré-condensateur (VK), les hydrocarbures à chaîne courte sortant de l'au moins un deuxième réacteur (R2) étant refroidis dans l'au moins un pré-condensateur (VK) ; et
f) l'introduction des hydrocarbures à chaîne courte refroidis dans l'au moins un pré-condensateur (VK) dans au moins un condensateur principal (HK), les hydrocarbures à chaîne courte sortant de l'au moins un pré-condensateur (VK) étant liquéfiés dans l'au moins un condensateur principal (HK).

2. Procédé selon la revendication 1, **caractérisé en ce que** des mélanges de polyoléfines constitués de polyéthylène (PE) et de polypropylène (PP) sont décomposés.

3. Procédé selon la revendication 1 ou 2, **caractérisé en ce que** les polyoléfines sont fondues dans au moins une extrudeuse (E), notamment dans une extrudeuse bivis, à des températures de jusqu'à 300 °C, notamment à des températures comprises entre 200 °C et 300 °C.

4. Procédé selon l'une quelconque des revendications précédentes, **caractérisé en ce qu'**une décomposition des polyoléfines ayant un poids moléculaire type de plus de $10^5$ kg/mol en oligomères ayant un poids moléculaire compris entre $10^2$ et $10^4$ kg/mol, de préférence $10^3$ et $10^4$ kg/mol, a lieu dans l'au moins un premier réacteur (R1).

5. Procédé selon l'une quelconque des revendications précédentes, **caractérisé en ce que** le mélange d'oligomères quittant l'au moins un premier réacteur (R1) est mélangé avec l'au moins un minéral argileux en tant que catalyseur de dépolymérisation avant l'introduction dans l'au moins un deuxième réacteur (R2).

6. Procédé selon l'une quelconque des revendications précédentes, **caractérisé en ce que** l'au moins un minéral argileux est choisi dans le groupe contenant les phyllosilicates, notamment la montmorillonite, l'illite, la bentonite, la kaolinite, la smectite, la chlorite, la vermiculite et le mica.

7. Procédé selon l'une quelconque des revendications précédentes, **caractérisé en ce que** le clivage des oligomères en hydrocarbures gazeux à chaîne courte ayant un poids moléculaire inférieur à 500 kg/mol a lieu dans l'au moins un deuxième réacteur (R2).

8. Unité pour la réalisation d'un procédé selon l'une quelconque des revendications précédentes, comprenant:

  - au moins une extrudeuse (E) pour la fusion des polyoléfines,
  - au moins un filtre à masse fondue (SF) agencé en aval de l'au moins une extrudeuse (E), pour la purification de la masse fondue de polyoléfines,
  - au moins un premier réacteur (R1) agencé en aval de l'au moins un filtre à masse fondue (SF), pour la décomposition des polyoléfines en oligomères,
  - au moins un deuxième réacteur (R2) agencé en aval de l'au moins un premier réacteur (R1), pour la décomposition des oligomères formés dans l'au moins un premier réacteur (R1) en hydrocarbures à chaîne courte,
  - au moins un pré-condensateur (VK) agencé en aval de l'au moins un deuxième réacteur (R2), pour le pré-refroidissement des hydrocarbures gazeux à chaîne courte quittant le deuxième réacteur (R2), et
  - au moins un condensateur principal (HK) agencé en aval de l'au moins un pré-condensateur (VK), pour la condensation des hydrocarbures à chaîne courte pré-refroidis dans l'au moins un pré-condensateur (VK).

9. Unité selon la revendication 8, **caractérisée en ce que** l'au moins un premier réacteur (R1) et l'au moins un deuxième réacteur (R2) disposent chacun d'au moins deux zones de chauffage réglables, de préférence d'au moins cinq zones de chauffage réglables.

10. Unité selon la revendication 8 ou 9, **caractérisée en ce que** l'au moins un premier réacteur (R1) et l'au moins un deuxième réacteur (R2) disposent chacun d'un organe d'agitation sous la forme d'un agitateur à hélice, d'un agitateur à ancre, d'une vis ou d'une combinaison de ceux-ci, de préférence sous la forme d'une combinaison d'un agitateur à hélice et d'une vis.

11. Unité selon l'une quelconque des revendications 8 à 10, **caractérisée en ce que** les parois intérieures et les composants internes de l'au moins un premier réacteur (R1) et/ou de l'au moins un deuxième réacteur (R2) comprennent au moins un revêtement en céramique.

12. Unité selon l'une quelconque des revendications 8 à 11, **caractérisée en ce que** l'au moins un premier réacteur (R1) et l'au moins un deuxième réacteur (R2) présentent chacun un rapport hauteur:diamètre (H:D) de 7:1, de préférence de 5,5:1.

13. Unité selon l'une quelconque des revendications 8 à 12, **caractérisée en ce que** l'au moins un pré-condensateur (VK) est raccordé avec au moins un contenant de mélange (M) destiné à recevoir les paraffines formées dans l'au moins un pré-condensateur (VK) pendant le pré-refroidissement des hydrocarbures à chaîne courte quittant le deuxième réacteur (R2).

14. Unité selon l'une quelconque des revendications 8 à 13, **caractérisée par** au moins une unité de commande pour la commande du taux d'introduction des polyoléfines dans ladite au moins une extrudeuse (E) et pour la commande de la température de l'unité.

15. Produit huileux fabriqué dans un procédé selon l'une quelconque des revendications 1 à 7, comprenant les fractions gaz liquide (C3-C6), essence (C7-C10), kérosène (C11-C13), gazole (C14-C19), gazole lourd (C20-C22) et paraffines/microcires (> C22).

```
┌────────────────────────┐        ┌──────────────────┐
│  Aufarbeitung PE/PP-    │ ────▶  │   Abfallstoffe   │
│       Gemisch          │        └──────────────────┘
└────────────────────────┘
            │
            ▼
┌────────────────────────┐
│     Dosiereinheit      │
└────────────────────────┘
            │
            ▼
┌────────────────────────┐
│      Extruder E        │
└────────────────────────┘
            │
            ▼                                        ┌──────────────┐
┌────────────────────────┐                           │    Fackel    │
│    Schmelzefilter SF   │                           └──────────────┘
└────────────────────────┘                                  ▲
            │                                                │
            ▼                                                │
┌────────────────────────┐                                  │
│    Schmelzepumpe SP     │                                  │
└────────────────────────┘                                  │
            │                                                │
            ▼                                                │
┌────────────────────────────────────────┐                  │
│        Rührkesselreaktor R1            │ ─────────────────┤
└────────────────────────────────────────┘                  │
      │              │                  │                    │
      │              ▼                  ▼                    │
┌────────────┐  ┌────────────┐   ┌──────────────────┐        │
│ Stickstoff-│  │Pufferbehälter│  │ Schmelzepumpe SP │        │
│Überlagerung│  └────────────┘   └──────────────────┘        │
└────────────┘         ▲                 │                    │
      │                │                 ▼                    │
      ▼                │         ┌──────────────────────────┐ │
┌────────────────────────────────────────┐                  │ │
│        Rührkesselreaktor R2            │ ◀──────┐         │ │
└────────────────────────────────────────┘        │         │ │
      │                                      ┌──────────────┐│ │
      ▼                                      │ Schmelzepumpe ││
┌────────────────────┐                       └──────────────┘│
│  Vorkondensator VK  │ ────▶ ┌──────────────┐    ▲           │
└────────────────────┘        │ Mischerbehälter│──┘           │
      │                       └──────────────┘                │
      ▼                                                        │
┌────────────────────────────────────────┐                    │
│         Hauptkondensator HK            │ ───────────────────┘
└────────────────────────────────────────┘
      │                        │
      ▼                        ▼
                    ┌──────────────────────────┐
                    │  Fraktionierte Destillation │
                    └──────────────────────────┘
┌──────────────────────────┐    │              │
│ Auffangbehälter Produktöle│   ▼              ▼
└──────────────────────────┘ ┌──────────┐  ┌──────────────┐
                             │Sammeltank │  │   Sammeltank  │
                             │   Öle     │  │    Benzin     │
                             └──────────┘  └──────────────┘
```

**Figur 1**

FIG 2A

Fackel

R1

R2

HK

VK

HZ5        HZ5

HZ4        HZ4

HZ3        HZ3

Z

E          HZ2        HZ2    SP

SP

SF  SP     HZ1        HZ1

SP

M

FIG 2B

**Figur 3**

**Figur 4**

**Figur 5**

**Figur 6**

FIG7A

Schmelze mit Bentonit-katalysator zum Reaktor R2

S

A A

Schmelze von Reaktor 1

Gemisch mit Bentonit-katalysator von Mischerbehälter

FIG7B
(A-A)

**EP 2 981 572 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4584421 A
- EP 1745115 B1
- DE 19722586 A1